# EUROPEAN PATENT SPECIFICATION

(11) **EP 2 858 658 B1**
(45) Date of publication and mention of the grant of the patent: **22.07.2020**
(21) Application number: 13799987.6
(22) Date of filing: 04.06.2013
(51) Int. Cl.: A61K 36/8964, A61K 36/756, A61K 36/744, A61K 36/718, A61K 36/489, A61K 36/42, A61K 36/29, A61K 36/185, A61P 1/00, A61K 31/4375, A61K 35/74, A61K 35/741, A61K 35/745, C12Q 1/68, A23L 33/00, A23L 33/105

(54) **METHODS AND COMPOSITIONS FOR IMPROVING GUT MICROBIOTA POPULATION**
VERFAHREN UND ZUSAMMENSETZUNGEN ZUR VERBESSERUNG DER DARM-BIOZÖNOSE
PROCÉDÉS ET COMPOSITIONS POUR L'AMÉLIORATION DE LA POPULATION DU MICROBIOTA DE L'INTESTIN

(30) Priority: 06.06.2012 CN 201210185004
(43) Date of publication of application: 15.04.2015
(73) Proprietor: Shanghai Jiao Tong University, Minhang District Shanghai 200240 (CN); Perfect (China) Co. Ltd, Guangdong 528420 (CN)
(72) Inventor: ZHAO, Liping, Shanghai 200240 (CN); ZHANG, Xu, Ottawa, ON K1H 8M5 (CA); ZHANG, Menghui, Shanghai 200240 (CN); ZHAO, Yufeng, Shanghai 200240 (CN); PANG, Xiaoyan, Shanghai 200240 (CN); ZHANG, Xiaojun, Shanghai 200240 (CN); WANG, Linghua, Shanghai 200240 (CN); NING, Guang, Shanghai 200025 (CN); LI, Xiaoying, Shanghai 200025 (CN); ZHANG, Yifei, Shanghai 200025 (CN)
(74) Representative: Haseltine Lake Kempner LLP
(86) International application number: PCT/CN2013/076709
(87) International publication number: WO 2013/182038

(56) References cited:
- CN-A- 101 291 597
- US-A1- 2011 281 852
- US-B1- 6 551 628
- DATABASE WPI Week 201244 Thomson Scientific, London, GB; AN 2012-B90995 XP002755592, & CN 102 327 309 A (UNIV CHENGDU TRADITIONAL CHINESE MEDICIN) 25 January 2012 (2012-01-25)
- DATABASE WPI Week 200974 Thomson Scientific, London, GB; AN 2009-P37360 XP002755593, & CN 101 524 404 A (YADA PHARM CO LTD ZIBO DEV ZONE) 9 September 2009 (2009-09-09)
- HAN JUNLING ET AL: "Modulating gut microbiota as an anti-diabetic mechanism of berberine.", MEDICAL SCIENCE MONITOR : INTERNATIONAL MEDICAL JOURNAL OF EXPERIMENTAL AND CLINICAL RESEARCH JUL 2011, vol. 17, no. 7, July 2011 (2011-07), pages RA164-RA167, XP009189163, ISSN: 1643-3750
- XIE, SHAN-SHAN ET AL.: "Effect of Huang-Lian-Jie-Du-Decoction on pharmacokinetics of verapamil in rats", JOURNAL OF PHARMACY AND PHARMACOLOGY, vol. 62, no. 4, April 2010 (2010-04), pages 440-447, XP009189161, DOI: 10.1211/JPP/62.04.0005
- THOMAS L V ET AL: "New insights into the impact of the intestinal microbiota on health and disease: A symposium report", BRITISH JOURNAL OF NUTRITION 2012 CAMBRIDGE UNIVERSITY PRESS GBR, vol. 107, no. SUPPL.1, January 2012 (2012-01), pages S1-S13, XP008157329, ISSN: 0007-1145
- ZHENG, JINBAO ET AL.: 'EFFECTS OF AQUEOUS EXTRACTS OF COPTIS CHINENSIS FRANCH AND PRUNUS UME ON INTESTINAL BACTERIA IN VITRO' FINE CHEMICALS vol. 25, no. 8, 31 August 2008, pages 758 - 761, 765, XP008175719
- SHI, XUEKUI ET AL.: 'The effects of boiling Rhizoma Coptidis on intestinal microflora of mouse.' JOURNAL OF MUDANJIANG MEDICAL COLLEGE vol. 22, no. 1, 28 February 2001, pages 10 - 12, XP008175743
- SHI, XUE KUI ET AL.: 'The effects of several boiling Chinese herbal medicine on intestinal microflora of mice' JOURNAL OF MUDANJIANG MEDICAL COLLEGE vol. 25, no. 6, 31 December 2004, pages 7 - 9, XP008175742

### SEQUENCE LISTING

<110> Shanghai Jiao Tong University
   PERFECT CHINA CO., LTD
<120> METHODS AND COMPOSITIONS FOR IMPROVING GUT MICROBIOTA POPULATION
<130> DAG12502P
<160> 268
<170> PatentIn version 3.5
<210> 1
   <211> 137
   <212> DNA
   <213> Helicobacter 16S rRNA gene V3 region
<400> 1
   tagggaatat tgctcaatgg gggaaaccct gaagcagcaa cgccgcgtgg aggatgaagg       60
   ttttcggatt gtaaactcct tttctaagag aagattatga cggtatctta ggaataagca      120
   ccggctaact ccgtgcc                                                     137
<210> 2
   <211> 116
   <212> DNA
   <213> Helicobacter 16S rRNA gene V3 region
<400> 2
   ggggaaaccc tgaagcagca acgccgcgtg gaggatgaag gtttcggatt gtaaactcct       60
   ttctaagaga agattatgac ggtatcttag gaataagcac cggctaactc cgtgcc          116
<210> 3
   <211> 163
   <212> DNA
   <213> Lawsonia 16S rRNA gene V3 region
<400> 3
   tggggaatat tgcgcaatgg gcgaaagcct gacgcagcga cgccgcgtga gggatgaagg 60
   tcctcggatc gtaaacctct gtcagggggg aagaagcgcc tgtgagcaaa tagttcatgg 120
   gtttgacggt acccccaaag gaagcaccgg ctaactccgt gcc 163
<210> 4
   <211> 157
   <212> DNA
   <213> Bacteroides 16S rRNA gene V3 region
<400> 4
   tgaggaatat tggtcaatgg gcgcaggcct gaaccagcca agtagcgtga aggatgactg 60
   ccctatgggt tgtaaacttc ttttatatgg gaataaagtt ttccacgtgt ggaattttgt 120
   atgtgccata tgaataagga tcggctaact ccgtgcc 157
<210> 5
   <211> 157
   <212> DNA
   <213> Bacteroides 16S rRNA gene V3 region
<400> 5
   tgaggaatat tggtcaatgg acgagagtct gaaccagcca agtagcgtga aggatgactg 60
   ccctatgggt tgtaaacttc ttttatatgg gaataaagtg agccacgtgt ggctttttgt 120
   atgtaccata cgaataagga tcggctaact ccgtgcc 157
<210> 6
   <211> 157
   <212> DNA
   <213> Bacteroides 16S rRNA gene V3 region
<400> 6
   tgaggaatat tggtcaatgg gcgcaggcct gaaccagcca agtagcgtga aggatgactg 60
   ccctatgggt tgtaaacttc ttttatatgg gaataaagtt ttccacgtgt ggaattttgt 120
   atgtaccata tgaataagga tcggctaact ccgtgcc 157
<210> 7
   <211> 159
   <212> DNA
   <213> Bacteroides 16S rRNA gene V3 region
<400> 7
   tgaggaatat tggtcaatgg gcgcaggcct gaaccagcca agtagcgtga aggatgactg 60
   ccctatgggt tgtaaacttc ttttatatgg gaataaagtt ttcctacgtg tggaattttg 120
   ttatgtacca tatgaataag gatcggctaa ctccgtgcc 159
<210> 8
   <211> 158
   <212> DNA
   <213> Prevotella 16S rRNA gene V3 region
<400> 8
   tgaggaatat tggtcaatgg gcgcgagcct gaaccagcca agtagcgtgc aggacgacgg 60
   ccctatgggt tgtaaactgt cttttatacg gggataaagt atgccacgtg tggtttattg 120
   caggtaccgt atgaataagg accggctaat tccgtgcc 158
<210> 9
   <211> 157
   <212> DNA
   <213> Prevotella 16S rRNA gene V3 region
<400> 9
   tgaggaatat tggtcaatgg gcgcgagcct gaaccagcca agtagcgtgc aggacgacgg 60
   ccctatgggt tgtaaactgc ttttatacgg ggataaagta tgccacgtgt ggtttattgc 120
   aggtaccgta tgaataagga ccggctaatt ccgtgcc 157
<210> 10
   <211> 161
   <212> DNA
   <213> Barnesiella 16S rRNA gene V3 region
<400> 10
   tgaggaatat tggtcaatgg gcggaagcct gaaccagcca agtcgcgtga gggaagacgg 60
   ccctacgggt tgtaaacctc ttttgccggg gaggcaatgc ccacgctcgc gagctgggaa 120
   ggagagtacc cggagaaaaa gacatcggct aactccgtgc c 161
<210> 11
   <211> 160
   <212> DNA
   <213> Barnesiella 16S rRNA gene V3 region
<400> 11
   tgaggaatat tggtcaatgg gcggaagcct gaaccagcca agtcgcgtga gggaagacgg 60
   ccctacgggt tgtaaacctc ttttgccggg gagcaatgcc cacgctcgcg agctgggaag 120
   gagagtaccc ggagaaaaag acatcggcta actccgtgcc 160
<210> 12
   <211> 160
   <212> DNA
   <213> Barnesiella 16S rRNA gene V3 region
<400> 12
   tgaggaatat tggtcaatgg gcggaagcct gaaccagcca agtcgcgtga gggaagacgg 60
   ccctacgggt tgtaaacctc ttttgccggg gagcaaatgc ccagctcgcg agctgggaag 120
   gagagtaccc ggagaaaaag catcggctaa actccgtgcc 160
<210> 13
   <211> 158
   <212> DNA
   <213> Butyricimonas 16S rRNA gene V3 region
<400> 13
   tgaggaatat tggtcaatgg gcgagagcct gaaccagcca agtcgcgtga gggaagacgg 60
   ccctacgggt tgtaaacctc ttttgtcagg gagcaagaac aggcacgtgt gcctgactga 120
   gagtacctga agaaaaagac atcggctaac tccgtgcc 158
<210> 14
   <211> 158
   <212> DNA
   <213> Porphyromonadaceae 16S rRNA gene V3 region
<400> 14
   tgaggaatat tggtcaatgg ccggaaggct gaaccagcca agtcgcgtga gggactaagg 60
   ccctacgggt cgtaaacctc ttttgccggg gagcaatggg gcccttgcga gggcccaggg 120
   agagtacccg gagaaaaagc atcggctaac tccgtgcc 158
<210> 15
   <211> 158
   <212> DNA
   <213> Barnesiella 16S rRNA gene V3 region
<400> 15
   tgaggaatat tggtcaatgg ccgggaggct gaaccagcca agtcgcgtga gggatgacgg 60
   ccctacgggt tgtaaacctc ttttgtcggg gagcaaagga cttcacgagt ggagtttcga 120
   gagtacccga agaaaaagac atcggctaac tccgtgcc 158
<210> 16
   <211> 157
   <212> DNA
   <213> Barnesiella 16S rRNA gene V3 region
<400> 16
   tgaggaatat tggtcaatgg ccgggaggct gaaccagcca agtcgcgtga gggatgacgg 60
   ccctacgggt tgtaaacctc ttttgtcggg gagcaaagga cttcacgagt ggagtttcga 120
   gagtacccga agaaaaagca tcggctaact ccgtgcc 157
<210> 17
   <211> 155
   <212> DNA
   <213> Porphyromonadaceae 16S rRNA gene V3 region
<400> 17
   tgaggaatat tggtcaatgg ccgggaggct gaaccagcca agtcgcgtga gggatgacgg 60
   ccctacgggt tgtaaacctc tttgtcgggg agcaaaggac ttcacgagtg gagtttcgag 120
   agtacccgaa gaaagacatc ggctaactcc gtgcc 155
<210> 18
   <211> 157
   <212> DNA
   <213> Butyricimonas 16S rRNA gene V3 region
<400> 18
   tgaggaatat tggtcaatgg gcgagagcct gaaccagcca agtcgcgtga gggaagacgg 60
   ccctacgggt tgtaaacctc ttttgtcagg gagcaagaac aggcacgtgt gcctgactga 120
   gagtacctga agaaaaagca tcggctaact ccgtgcc 157
<210> 19
   <211> 157
   <212> DNA
   <213> Barnesiella 16S rRNA gene V3 region
<400> 19
   tgaggaatat tggtcaatgg ccgagaggct gaaccagcca agtcgcgtga gggatgacgg 60
   ccctacgggt tgtaaacctc ttttgtcggg gagcaaagga cttcacgtgt gaagtttcga 120
   gagtacccga agaaaaagca tcggctaact ccgtgcc 157
<210> 20
   <211> 158
   <212> DNA
   <213> Porphyromonadaceae 16S rRNA gene V3 region
<400> 20
   tgaggaatat tggtcaatgg gcggaagcct gaaccagcca agtcgcgtga gggaagacgg 60
   tcctatggat tgtaaacctc ttttgccggg gagcaatgcc gctcttgcga gagcggaggg 120
   agagtacccg gagaaaaagc atcggctaac tccgtgcc 158
<210> 21
   <211> 158
   <212> DNA
   <213> Barnesiella 16S rRNA gene V3 region
<400> 21
   tgaggaatat tggtcaatgg ccggaaggct gaaccagcca agtcgcgtga gggaataagg 60
   ccctacgggt cgtaaacctc ttttgtcagg gagcaaagct ggctacgcgt agccagaagg 120
   agagtacctg aagaaaaagc atcggctaac tccgtgcc 158
<210> 22
   <211> 156
   <212> DNA
   <213> Porphyromonadaceae 16S rRNA gene V3 region
<400> 22
   tgaggaatat tggtcaatgg gcggaagcct gaaccagcca agtcgcgtga gggaagacgg 60
   ccctacgggt tgtaaacctc tttgccggga gcaatgccca gctcgcgagc tgggaaggag 120
   agtacccgga gaaaagacat cggctaactc cgtgcc 156
<210> 23
   <211> 157
   <212> DNA
   <213> Barnesiella 16S rRNA gene V3 region
<400> 23
   tgaggaatat tggtcaatgg gcggaagcct gaaccagcca agtcgcgtga gggaagacgg 60
   ccctacgggt tgtaaacctc tttgccgggg agcaatgccc agctcgcgag ctgggaagga 120
   gagtacccgg agaaaagaca tcggctaact ccgtgcc 157
<210> 24
   <211> 158
   <212> DNA
   <213> Porphyromonadaceae 16S rRNA gene V3 region
<400> 24
   tgaggaatat tggtcaatgg gcggaagcct gaaccagcca agtcgcgtga gggactaagg 60
   ccctacgggt cgtaaacctc ttttgccggg gagcaagccg tcccacgtgt gggccggtgg 120
   agagtacccg gagaaaaagc atcggctaac tccgtgcc 158
<210> 25
   <211> 159
   <212> DNA
   <213> Porphyromonadaceae 16S rRNA gene V3 region
<400> 25
   tgaggaatat tggtcaatgg gcggaagcct gaaccagcca agtcgcgtga gggactaagg 60
   ccctacgggt cgtaaacctc ttttgccggg gagcaagccg tcccacgtgt gggccggtgg 120
   agagtacccg gagaaaaaga catcggctaa ctccgtgcc 159
<210> 26
   <211> 158
   <212> DNA
   <213> Barnesiella 16S rRNA gene V3 region
<400> 26
   tgaggaatat tggtcaatgg gcggaagcct gaaccagcca agtcgcgtga gggaagacgg 60
   ccctacgggt tgtaaacctc ttttgccggg gagcaatgcc cagctcgcga gctgggaagg 120
   agagtacccg gagaaaaagc atcggctaac tccgtgcc 158
<210> 27
   <211> 161
   <212> DNA
   <213> Barnesiella 16S rRNA gene V3 region
<400> 27
   tgaggaatat tggtcaatgg gcggaagcct gaaccagcca agtcgcgtga gggaagacgg 60
   ccctacgggt tgtaaacctc tttttgccgg gggagcaatg cccagctcgc gagctgggaa 120
   ggagagtacc cggagaaaaa agcatcggct aactccgtgc c 161
<210> 28
   <211> 157
   <212> DNA
   <213> Parabacteroides 16S rRNA gene V3 region
<400> 28
   tgaggaatat tggtcaatgg gcgagagcct gaaccagcca agtcgcgtga aggatgaagg 60
   atctatggtt tgtaaacttc ttttatatgg gaataaagtg aggaacgtgt tcctttttgt 120
   atgtaccata tgaataagca tcggctaact ccgtgcc 157
<210> 29
   <211> 157
   <212> DNA
   <213> Bacteroides 16S rRNA gene V3 region
<400> 29
   tgaggaatat tggtcaatgg acgagagtct gaaccagcca agtagcgtga aggatgactg 60
   ccctatgggt tgtaaacttc ttttatacgg gaataaagtg aggcacgtgt gcctttttgt 120
   atgtaccgta tgaataagga tcggctaact ccgtgcc 157
<210> 30
   <211> 158
   <212> DNA
   <213> Alistipes 16S rRNA gene V3 region
<400> 30
   tgaggaatat tggtcaatgg acgcaagtct gaaccagcca tgccgcgtgc aggaagacgg 60
   ctctatgagt tgtaaactgt cttttgtact agggtaaacg ctcttacgtg taggagcctg 120
   aaagtatagt acgaataagg atcggctaac tccgtgcc 158
<210> 31
   <211> 157
   <212> DNA
   <213> Alistipes 16S rRNA gene V3 region
<400> 31
   tgaggaatat tggtcaatgg acgcaagtct gaaccagcca tgccgcgtgc aggaagacgg 60
   ctctatgagt tgtaaactgc ttttgtacta gggtaaacgc ttctacgtgt aggagcctga 120
   aagtatagta cgaataagga tcggctaact ccgtgcc 157
<210> 32
   <211> 137
   <212> DNA
   <213> Coriobacteriaceae 16S rRNA gene V3 region
<400> 32
   tggggaattt tgcgcaatgg ggggaaccct gacgcagcaa cgccgcgtgc gggatgacgg 60
   ccctcgggtt gtaaaccgct ttcagcaggg aagaccacga cggtacctgc agaagaagct 120
   ccggctaact acgtgcc 137
<210> 33
   <211> 157
   <212> DNA
   <213> Flavobacteriaceae 16S rRNA gene V3 region
<400> 33
   tgaggaatat tgggcaatgg gcggaagcct gacccagcca tgccgcgtgc aggaagacag 60
   ccctatgggt cgtaaactgc ttttttagag gaagaataaa gtctacgtgt agaccgatga 120
   cggtacttta agaaaaagca tcggctaact ccgtgcc 157
<210> 34
   <211> 138
   <212> DNA
   <213> Blautia 16S rRNA gene V3 region
<400> 34
   gtagggaata ttgcacaatg ggggaaaccc tgatgcagcg acgccgcgtg aaggaagaag 60
   tatctcggta tgtaaacttc tatcagcagg gaagacaatg acggtacctg actaagaagc 120
   cccggctaac tacgtgcc 138
<210> 35
   <211> 137
   <212> DNA
   <213> Hespellia 16S rRNA gene V3 region
<400> 35
   tggggaatat tgcacaatgg gggaaaccct gatgcagcga cgccgcgtga aggaagaagt 60
   atttcggtat gtaaacttct atcagcaggg aagaaagtga cagtacctga ctaagaagcc 120
   ccggctaact acgtgcc 137
<210> 36
   <211> 140
   <212> DNA
   <213> Blautia 16S rRNA gene V3 region
<400> 36
   tggggaatat tgcacaatgg gggaaacccg tacgatgcag cgacgccgcg tgaaggaaga 60
   agtatctcgg tatgtaaact tctatcagca gggaagataa tgacggtacc tgactaagaa 120
   gccccggcta actacgtgcc 140
<210> 37
   <211> 142
   <212> DNA
   <213> Clostridiales 16S rRNA gene V3 region
<400> 37
   tggggaatat tgcacaatgg gggaaacccg tacgatgcag cgacgccgcg tgaaggaaga 60
   agtatctcgg tatgtaaact tactatcagc agggaagata atgacggtac ctgactaaga 120
   agccccggcc taactacgtg cc 142
<210> 38
   <211> 114
   <212> DNA
   <213> Blautia 16S rRNA gene V3 region
<400> 38
   aaaccctgat gcagcgacgc cgcgtgaagg aagaagtatc tcggtatgta aacttctatc 60
   agcagggaag acaatgacgg tacctgacta agaacgcccg gctaactacg tgcc 114
<210> 39
   <211> 137
   <212> DNA
   <213> Blautia 16S rRNA gene V3 region
<400> 39
   tggggaatat tgcacaatgg gggaaaccct gatgcagcga cgccgcgtga aggaagaagt 60
   atctcggtat gtaaacttct atcagcaggg aagataatga cggtacctga ctaagaagcc 120
   ccggctaatt acgtgcc 137
<210> 40
   <211> 137
   <212> DNA
   <213> Blautia 16S rRNA gene V3 region
<400> 40
   tggggaatat tgcacaatgg gggaaaccct gatgcagcga cgccgcgtga aggaagaagt 60
   atctcggtat gtaaacttct atcagcaggg aagataatga cggtacctga ctaagaagcc 120
   ccggctaact acgtgcc 137
<210> 41
   <211> 138
   <212> DNA
   <213> Clostridiales 16S rRNA gene V3 region
<400> 41
   tggggaatat tgcacaatgg gggaaaccct gatgcagcaa cgccgcgtgg gtgaaggagc 60
   gtttcggcgc gtaaagccct gtcagcgggg aagaaaaaag acggtacccg accaagaagc 120
   cccggctaac tacgtgcc 138
<210> 42
   <211> 137
   <212> DNA
   <213> Lachnospiraceae 16S rRNA gene V3 region
<400> 42
   tggggaatat tgcacaatgg gcgcaagcct gatgcagcga cgccgcgtga gtgaagaagt 60
   atttcggtat gtaaagctct atcagcaggg aagaaaatga cggtacctga ctaagaagcc 120
   ccggctaact acgtgcc 137
<210> 43
   <211> 137
   <212> DNA
   <213> Lachnospiraceae 16S rRNA gene V3 region
<400> 43
   tggggaatat tgcacaatgg gggaaaccct gatgcagcga cgccgcgtga aagatgaagt        60
   atttcggtat gtaaacttct atcagcaggg aagaaaatga cggtacctga ctaagaagcc       120
   ccggctaact acgtgcc                                                      137
<210> 44
   <211> 137
   <212> DNA
   <213> Lachnospiraceae 16S rRNA gene V3 region
<400> 44
   tggggaatat tgcacaatgg aggaaactct gatgcagcga cgccgcgtga gtgaagaagt        60
   atttcggtat gtaaagctct atcagcaggg aagacagtga cggtacctga ctaagaagct       120
   ccggctaaat acgtgcc                                                      137
<210> 45
   <211> 138
   <212> DNA
   <213> Lachnospiraceae 16S rRNA gene V3 region
<400> 45
   tggggaatat tgcacaatgg ggggaaccct gatgcagcga cgccgcgtga gtgacgaagt        60
   atctcggtat gtaaagctct gtcagcaggg aagaagaatg acggtacctg aagaagaagc       120
   accggctaaa tacgtgcc                                                     138
<210> 46
   <211> 137
   <212> DNA
   <213> Lachnospiraceae 16S rRNA gene V3 region
<400> 46
   tggggaatat tgcacaatgg gcgaaagcct gatgcagcga cgccgcgtga gtgaagaagt        60
   atttcggtat gtaaagctct atcagcaggg aagaaaatga cggtacctga ctaagaagcc       120
   ccggctaact acgtgcc                                                      137
<210> 47
   <211> 140
   <212> DNA
   <213> Clostridiales 16S rRNA gene V3 region
<400> 47
   tggggaatat tgcacaatgg gcgaaagcct gatgcagcaa cgccgcgtga aggaagacgg 60
   ttttcggatt gtaaacttct atcaataggg aagaaagaaa tgacggtacc taaataagaa 120
   gccccggcta actacgtgcc 140
<210> 48
   <211> 140
   <212> DNA
   <213> Clostridiales 16S rRNA gene V3 region
<400> 48
   tggggaatat tgcacaatgg gggaaaccct gatgcagcaa cgccgcgtga aggaagacgg 60
   ttttcggatt gtaaacttct atcaataggg aagaaagaaa tgacggtacc taaataagaa 120
   gccccggcta actacgtgcc 140
<210> 49
   <211> 137
   <212> DNA
   <213> Clostridiales 16S rRNA gene V3 region
<400> 49
   tggggaatat tgcacaatgg gggaaaccct gatgcagcga cgccgcgtgg aggaagaagg 60
   tcttcggatt gtaaactcct gtcccagggg acgataatga cggtaccctg ggaggaagca 120
   ccggctaact acgtgcc 137
<210> 50
   <211> 138
   <212> DNA
   <213> clostridiales 16S rRNA gene V3 region
<400> 50
   tggggaatat tgcacaatgg gcgcaagcct gatgcagcaa cgccgcgtga aggaagacgg 60
   ttttcggatt gtaaacttct gttcttagtg aagaagaatg acggtagcta aggagcaagc 120
   cacggctaac tacgtgcc 138
<210> 51
   <211> 138
   <212> DNA
   <213> Clostridiales 16S rRNA gene V3 region
<400> 51
   tggggaatat tgcacaatgg gcgcaagcct gatgcagcaa cgccgcgtga aggaagacgg 60
   ttttcggatt gtaaacttct gttcttagtg aagaataatg acggtaacta aggagcaagc 120
   cacggctaac tacgtgcc 138
<210> 52
   <211> 137
   <212> DNA
   <213> Sedimentibacter 16S rRNA gene V3 region
<400> 52
   tggggaatat tgcacaatgg gggaaaccct gatgcagcga cgccgagtga gggaagaagg 60
   ttttcggatt gtaaacctct gtccttggtg aagataatga cggtagccaa ggaggaagct 120
   acggctaact acgtgcc 137
<210> 53
   <211> 140
   <212> DNA
   <213> Clostridiales 16S rRNA gene V3 region
<400> 53
   tgggggatat tgcacaatgg gggaaaccct gatgcagcga cgccgcgtga gggaagacgg 60
   ccttcgggtt gtaaacctct gtcgcagggg acgaaggaag tgacggtacc ctgtgaggaa 120
   gccacggcta actacgtgcc 140
<210> 54
   <211> 136
   <212> DNA
   <213> Ruminococcaceae 16S rRNA gene V3 region
<400> 54
   gaatattgcg caatgggggc aaccctgacg cagcaacgcc gcgtgaagga tgaaggtttt 60
   cggattgtaa acttctttta tcaaggacga aggacgtgac ggtacttgat gaataagcca 120
   cggctaacta cgtgcc 136
<210> 55
   <211> 141
   <212> DNA
   <213> Ruminococcaceae 16S rRNA gene V3 region
<400> 55
   tggggaatat tgggcaatgg gggaaaccct gacccagcaa cgccgcgtga aggaagaagg 60
   ctttcgggtt gtaaacttct tttaccaggg gacgaaggac gtgacggtac ctggagaaaa 120
   agcaacggct aactacgtgc c 141
<210> 56
   <211> 141
   <212> DNA
   <213> Ruminococcaceae 16S rRNA gene V3 region
<400> 56
   tggggaatat tgggcaatgg gggaaaccct gacccagcaa cgccgcgtga aggaagaagg 60
   ctttcgggtt gtaaacttct ttacctaggg acgaaggacg tgacggtacc tggagaaaaa 120
   gacaacggct aactacgtgc c 141
<210> 57
   <211> 140
   <212> DNA
   <213> Ruminococcaceae 16S rRNA gene V3 region
<400> 57
   tggggaatat tgggcaatgg gggaaaccct gacccagcaa cgccgcgtga aggaagaagg 60
   ttttcgggtt gtaaacttct tttaccaggg acgaaggacg tgacggtacc tggagaaaaa 120
   gcaacggcta actacgtgcc 140
<210> 58
   <211> 114
   <212> DNA
   <213> Clostridiales 16S rRNA gene V3 region
<400> 58
   gacccagcaa cgccgcgtga aggaagaagg tcgtttcggg ttagtaaact tcttttaccg 60
   agggacgaag gacgtgacgg tacctggaga aaaagcaacg gctaactacg tgcc 114
<210> 59
   <211> 140
   <212> DNA
   <213> Ruminococcaceae 16S rRNA gene V3 region
<400> 59
   tggggaatat tgggcaatgg gggaaaccct gacccagcaa cgccgcgtga aggaagaagg 60
   ctttcgggtt gtaaacttct tttaccaggg acgaaggacg tgacggtacc tggagaaaaa 120
   gcaacggcta actacgtgcc 140
<210> 60
   <211> 142
   <212> DNA
   <213> Ruminococcaceae 16S rRNA gene V3 region
<400> 60
   tggggaatat tgggcaatgg gggaaaccct gacccagcaa cgccgcgtga aggaagaagg 60
   ctttcgggtt gtaaacttct tttacctagg gacgaaggac gtgacggtac ctggagaaaa 120
   agacaacggc taactacgtg cc 142
<210> 61
   <211> 140
   <212> DNA
   <213> Ruminococcaceae 16S rRNA gene V3 region
<400> 61
   tggggaatat tgggcaatgg gggaaaccct gacccagcaa cgccgcgtga aggaagaagg 60
   ctttcgggtt gtaaacttct tttaccaggg acgaaggacg tgacggtacc tggagaaaag 120
   acaacggcta actacgtgcc 140
<210> 62
   <211> 141
   <212> DNA
   <213> Oscillibacter 16S rRNA gene V3 region
<400> 62
   tggggaatat tgggcaatgg gcgcaagcct gacccagcaa cgccgcgtga aggaagaagg 60
   ctttcgggtt gtaaacttct tttctggggg acgaagaaag tgacggtacc ccacggaata 120
   agccacggct aactacgtgc c 141
<210> 63
   <211> 140
   <212> DNA
   <213> Ruminococcaceae 16S rRNA gene V3 region
<400> 63
   tggggaatat tgggcaatgg gcgcaagcct gacccagcaa cgccgcgtga aggaagaagg 60
   ctttcgggtt gtaaacttct tttctggggg gcgaagaaag tgacggtacc ccaggaataa 120
   gccacggcta actacgtgcc 140
<210> 64
   <211> 140
   <212> DNA
   <213> Oscillibacter 16S rRNA gene V3 region
<400> 64
   tggggaatat tgggcaatgg gcgcaagcct gacccagcaa cgccgcgtga aggaagaagg 60
   ctttcgggtt gtaaacttct tttctggggg acgaagaaag tgacggtacc ccaggaataa 120
   gccacggcta actacgtgcc 140
<210> 65
   <211> 139
   <212> DNA
   <213> Ruminococcaceae 16S rRNA gene V3 region
<400> 65
   tggggaatat tgggcaatgg gcgcaagcct gacccagcaa cgccgcgtga aggaagaagg 60
   ctttcgggtt gtaaacttct tttctcgggg acgaacaaat gacggtaccc gaggaataag 120
   ccacggctaa ctacgtgcc 139
<210> 66
   <211> 139
   <212> DNA
   <213> Butyricicoccus 16S rRNA gene V3 region
<400> 66
   tggggaatat tgcgcaatgg gggcaaccct gacgcagcaa cgccgcgtga ttgaagaagg 60
   tcttcggatt gtaaaaatct tttatcaagg acgaagaagt gacggtactt gatgaataag 120
   ctccggctaa ctacgtgcc 139
<210> 67
   <211> 139
   <212> DNA
   <213> unknown bacterium 16S rRNA gene V3 region
<400> 67
   tggggaatat tgggcaatgg ggggaaccct gacccagcaa cgccgcgtga gggaagaagg 60
   ttttcggatc gtaaacctct gtccttggtg aagaggagaa gacggtagcc aaggaggaag 120
   ccccggctaa ctacgtgcc 139
<210> 68
   <211> 139
   <212> DNA
   <213> unknown bacterium 16S rRNA gene V3 region
<400> 68
   tggggaatat tgggcaatgg gggaaaccct gacccagcaa cgccgcgtga gggaagaagg 60
   ttttcggatc gtaaacctct gtccttggtg aagagaagaa gacggtagcc aaggaggaag 120
   ccccggctaa ctacgtgcc 139
<210> 69
   <211> 137
   <212> DNA
   <213> Dorea 16S rRNA gene V3 region
<400> 69
   tggggaatat tgcacaatgg gcgaaagcct gatgcagcaa cgccgcgtga aggatgaagt 60
   atttcggtat gtaaacttct atcagcaggg aagataacga cggtacctga ctaagaagcc 120
   ccggctaact acgtgcc 137
<210> 70
   <211> 140
   <212> DNA
   <213> Clostridiales 16S rRNA gene V3 region
<400> 70
   tggggaatat tgcacaatgg gcgcaagcct gatgcagcaa cgccgcgtga acgaagaagg 60
   tcttcggatt gtaaagttct gtccttaggg aagaagaaag tgacggtacc taaggaggaa 120
   gccccggcta actacgtgcc 140
<210> 71
   <211> 109
   <212> DNA
   <213> Lachnospiraceae 16S rRNA gene V3 region
<400> 71
   tggggaatat tgcacaatgg gcgaaagcct gatgcagcga cgccgcgtga gtgaagaagt 60
   atttcggtat gtaaagctct atcagcaggg aagaaaatga cggtacctg 109
<210> 72
   <211> 137
   <212> DNA
   <213> Clostridiales 16S rRNA gene V3 region
<400> 72
   tggggaatat tgcacaatgg gggaaaccct gatgcagcga cgccgcgtga aggaagaagt 60
   atttcggtat gtaaacttct atcagcaggg aagaaaatga cggtacctga ctaagaagcc 120
   ccggctaatt acgtgcc 137
<210> 73
   <211> 163
   <212> DNA
   <213> Allobaculum 16S rRNA gene V3 region
<400> 73
   tagggaattt ttcgtcaatg ggcgcaagcc tgaacgagca atgccgcgtg ggcgaagaag 60
   gtcttcggat cgtaaaactc tgttgcgggg gaaaaaggaa gggaagagga aatgcttttc 120
   ttttgatggt accccgccag aaagtcacgg ctaactacgt gcc 163
<210> 74
   <211> 163
   <212> DNA
   <213> Allobaculum 16S rRNA gene V3 region
<400> 74
   tagggaattt ttcgtcaatg ggcgcaagcc tgaacgagca atgccgcgtg agcgaagaag 60
   gtcttcggat cgtaaaactc tgttgcgggg gaaaaaggaa ggaaagagga aatgcttttc 120
   ttttgatggt accccgccag aaagtcacgg ctaactacgt gcc 163
<210> 75
   <211> 164
   <212> DNA
   <213> Allobaculum 16S rRNA gene V3 region
<400> 75
   tagggaattt tcgtcaatgg gcgcaagcct gaacgagcaa tgccgcgtgg gcgaagaagg 60
   tcttcggatc gtaaaaactc tgttgcgggg gaaaaaagga agggaagagg aaatgctttt 120
   cttttgatgg taccccgcca gaaagtcacg gctaactacg tgcc 164
<210> 76
   <211> 164
   <212> DNA
   <213> Allobaculum 16S rRNA gene V3 region
<400> 76
   tagggaattt tcgtcaatgg gcgcaagcct gaacgagcaa tgccgcgtgg gcgaagaagg 60
   tcttcggatc gtaaaactct gtttgcgggg ggaaaaagga agggaagagg aaatgctttt 120
   cttttgatgg taccccgcca gaaagtcacg gctaactacg tgcc 164
<210> 77
   <211> 162
   <212> DNA
   <213> Allobaculum 16S rRNA gene V3 region
<400> 77
   tagggaattt tcgtcaatgg gcgcaagcct gaacgagcaa tgccgcgtgg gcgaagaagg 60
   tcttcggatc gtaaaactct gttgcggggg aaaaaggaag ggaagaggaa atgcttttct 120
   tttgatggta ccccgccaga aagtcacggc taactacgtg cc 162
<210> 78
   <211> 161
   <212> DNA
   <213> Allobaculum 16S rRNA gene V3 region
<400> 78
   tagggaattt tcgtcaatgg gcgcaagcct gaacgagcaa tgccgcgtga gcgaagaagg 60
   tcttcggatc gtaaaactct gttgcgggga aaaggaaggg aagaggaaat gcttttcttt 120
   tgtatggtac cccgccagaa agtcacggct aactacgtgc c 161
<210> 79
   <211> 162
   <212> DNA
   <213> Allobaculum 16S rRNA gene V3 region
<400> 79
   tagggaattt tcgtcaatgg gcgcaagcct gaacgagcaa tgccgcgtgg gcgaagaagg 60
   tcttcggatc gtaaaactct gttgcggggg aaaaggaagg gaagaggaaa tgcttttctt 120
   ttgtatggta ccccgccaga aagtcacggc taactacgtg cc 162
<210> 80
   <211> 161
   <212> DNA
   <213> Allobaculum 16S rRNA gene V3 region
<400> 80
   tagggaattt tcgtcaatgg gcgcaagcct gaacgagcaa tgccgcgtgg gcgaagaagg 60
   tcttcggatc gtaaaactct gttgcggggg aaaaggaagg gaagaggaaa tgcttttctt 120
   ttgatggtac cccgccagaa agtcacggct aactacgtgc c 161
<210> 81
   <211> 159
   <212> DNA
   <213> Allobaculum 16S rRNA gene V3 region
<400> 81
   ggtaattttc gtcaatgggc gcaagcctga acgagcaatg ccgcgtgggc gaagaaggtc 60
   ttcggatacg taaactctgt tgcgggggaa aaaggaaggg aagaggaaat gctttctttt 120
   gatggtaccc cgccagaaag tcacggctaa ctacgtgcc 159
<210> 82
   <211> 163
   <212> DNA
   <213> Allobaculum 16S rRNA gene V3 region
<400> 82
   tagggaattt tcgtcaatgg gcgcaagcct gaacgagcaa tgccgcgtga gcgaagaagg 60
   tcttcggatc gtaaaactct gttgcggggg aaaaaggaag ggaagaggaa atgcttttct 120
   tttgatggta ccccgcccag aaagtcacgg ctaactacgt gcc 163
<210> 83
   <211> 163
   <212> DNA
   <213> Allobaculum 16S rRNA gene V3 region
<400> 83
   tagggaattt tcgtcaatgg gcgcaagcct gaacgagcaa tgccgcgtga gcgaagaagg 60
   tcttcggatc gtaaaactct gttgcggggg aaaaaaggaa gggaagagga aatgcttttc 120
   ttttgatggt accccgccag aaagtcacgg ctaactacgt gcc 163
<210> 84
   <211> 162
   <212> DNA
   <213> Allobaculum 16S rRNA gene V3 region
<400> 84
   tagggaattt tcgtcaatgg gcgcaagcct gaacgagcaa tgccgcgtga gcgaagaagg 60
   tcttcggatc gtaaaactct gttgcggggg aaaaaggaag ggaagaggaa atgcttttct 120
   tttgatggta ccccgccaga aagtcacggc taactacgtg cc 162
<210> 85
   <211> 163
   <212> DNA
   <213> Allobaculum 16S rRNA gene V3 region
<400> 85
   tagggaattt ttcgtcaatg ggcgcaagcc tgaacgagca atgccgcgtg aacgaggaag 60
   gtcttcggat cgtaaagttc tgttgagagg gaaaaagggt caccagagga aatgctggtg 120
   aagtgatatt acctttcgag gaagtcacgg ctaactacgt gcc 163
<210> 86
   <211> 162
   <212> DNA
   <213> Allobaculum 16S rRNA gene V3 region
<400> 86
   tagggaattt tcgtcaatgg gcgcaagcct gaacgagcaa tgccgcgtga acgaggaagg 60
   tcttcggatc gtaaagttct gttgagaggg aaaaagggtc accagaggaa atgctggtga 120
   agtgatatta cctttcgagg aagtcacggc taactacgtg cc 162
<210> 87
   <211> 160
   <212> DNA
   <213> Allobaculum 16S rRNA gene V3 region
<400> 87
   ggtaattttc gtcaatgggc gcaagcctga acgagcaatg ccgcgtgaac gaggaaggtc      60
   ttcggatcgt aaagttctgt tgagagggaa aaagggtcac cagaggaaat gctggtgaag     120
   tgatattacc tttcgaggaa gtcacggcta actacgtgcc                           160
<210> 88
   <211> 163
   <212> DNA
   <213> Allobaculum 16S rRNA gene V3 region
<400> 88
   tagggaattt tcgtcaatgg gcgcaagcct gaacgagcaa tgccgcgtga acgaggaagg      60
   tcttcggatc gtaaagttct gttgagaggg aaaaaagggt caccagagga aatgctggtg     120
   aagtgatatt acctttcgag gaagtcacgg ctaactacgt gcc                       163
<210> 89
   <211> 161
   <212> DNA
   <213> Allobaculum 16S rRNA gene V3 region
<400> 89
   tagggaattt tcgtcaatgg gcgcaagcct gaacgagcaa tgccgcgtga acgaggaagg      60
   tcttcggatc gtaaagttct gttgagaggg aaaagggtca ccagaggaaa tgctggtgaa     120
   gtgatattac ctttcgagga agtcacggct aactacgtgc c                         161
<210> 90
   <211> 163
   <212> DNA
   <213> Allobaculum 16S rRNA gene V3 region
<400> 90
   tagggaattt tcgtcaagtg ggcgcaagcc tgaacgagca atgccgcgtg aacgaggaag      60
   gtcttcggat acgtaaagtt ctgttgagag gaaaaagggt caccagagga aatgctggtg     120
   aagtgatatt acctttcgag gaagtcacgg ctaactacgt gcc                       163
<210> 91
   <211> 161
   <212> DNA
   <213> Allobaculum 16S rRNA gene V3 region
<400> 91
   tagggaattt tcgtcaatgg gcgcaagcct gaacgagcaa tgccgcgtga gcgaagaagg 60
   tcttcggatc gtaaaactct gttgcggggg aaaaggaagg gaagaggaaa tgcttttctt 120
   ttgatggtac cccgccagaa agtcacggct aactacgtgc c 161
<210> 92
   <211> 162
   <212> DNA
   <213> Holdemania 16S rRNA gene V3 region
<400> 92
   tagggaattt tcggcaatgg gcgaaagcct gaccgagcaa cgccgcgtga gtgaagaagg 60
   ccttcgggtt gtaaagctct gttgtgaagg aagaacggct catagaggga atgctatggg 120
   agtgacggta ctttaccaga aagccacggc taactacgtg cc 162
<210> 93
   <211> 163
   <212> DNA
   <213> Phascolarctobacterium 16S rRNA gene V3 region
<400> 93
   tggggaatct tccgcaatgg acgaaagtct gacggagcaa cgccgcgtga gtgatgaagg 60
   atttcggtct gtaaagctct gttgtttatg acgaacgtgc agtgtgtgaa caatgcattg 120
   caatgacggt agtaaacgag gaagccacgg ctaactacgt gcc 163
<210> 94
   <211> 137
   <212> DNA
   <213> Alphaaproteobacteria 16S rRNA gene V3 region
<400> 94
   tgaggaatat tgggcaatgg gggcaaccct gacccagcca tgccgcgtga gtgaagaagg 60
   ttttcggatt gtaaagctct ttcggatgtg acgatgatga cggtagcatc taaagaagcc 120
   ccggcaaact tcgtgcc 137
<210> 95
   <211> 117
   <212> DNA
   <213> Helicobacter 16S rRNA gene V3 region
<220>
   <221> misc_feature
   <222> 2..2
   <223> n is a, c, g, or t
<400> 95
   gngggaaacc ctgaagcagc aacgccgcgt ggaggatgaa ggtttcggat tgtaaactcc 60
   tttgttagag aagataatga cggtatctaa cgaataagca ccggctaact ccgtgcc 117
<210> 96
   <211> 137
   <212> DNA
   <213> Helicobacter 16S rRNA gene V3 region
<400> 96
   tagggaatat tgctcaatgg gggaaaccct gaagcagcaa cgccgcgtgg aggatgaagg 60
   ttttcggatt gtaaactcct tttgttagag aagataatga cggtatctaa cgaataagca 120
   ccggctaact ccgtgcc 137
<210> 97
   <211> 157
   <212> DNA
   <213> Prevotella 16S rRNA gene V3 region
<400> 97
   tgaggaatat tggtcaatgg gcggaagcct gaaccagcca agtagcgtgc aggaagacgg 60
   ccctacgggt tgtaaactgc ttttatgcgg ggataaagtg caatacgtgt attgctttgc 120
   aggtaccgca tgaataagga ccggctaatt ccgtgcc 157
<210> 98
   <211> 157
   <212> DNA
   <213> Prevotella 16S rRNA gene V3 region
<400> 98
   tgaggaatat tggtcaatgg gcggaagcct gaaccagcca agtagcgtgc aggatgacgg 60
   ccctacgggt tgtaaactgc ttttttgcgg gaataaagcg gctcacgtgt gagcctttgc 120
   atgtaccgca cgaataagga ccggctaatt ccgtgcc 157
<210> 99
   <211> 157
   <212> DNA
   <213> Prevotella 16S rRNA gene V3 region
<400> 99
   tgaggaatat tggtcaatgg gcgagagcct gaaccagcca agtagcgtgc aggatgacgg 60
   ccctatgggt tgtaaactgc ttttatacgg ggataaagtt ggggacgtgt ccccatttgt 120
   aggtaccgta tgaataagga ccggctaatt ccgtgcc 157
<210> 100
   <211> 159
   <212> DNA
   <213> Prevotellaceae 16S rRNA gene V3 region
<400> 100
   tgaggaatat tggtcaatgg tcgtgagact gaaccagcca agtagcgtgc gggatgaagg 60
   ccctccgggt cgtaaaccgc ttttagacgg ggataaaagg gcatacgtgt atgccgtatt 120
   gcatgtaccg tcagaaaaag gaccggctaa ttccgtgcc 159
<210> 101
   <211> 157
   <212> DNA
   <213> Bacteroidales 16S rRNA gene V3 region
<400> 101
   tgaggaatat tggtcaatgg gcgagagcct gaaccagcca agtagcgtga aggaagactg 60
   ccctatgggt tgtaaacttc ttttataagg gaataaagag cgccacgtgt ggtgtgttgt 120
   atgtacctta tgaataagca tcggctaatt ccgtgcc 157
<210> 102
   <211> 157
   <212> DNA
   <213> Bacteroidales 16S rRNA gene V3 region
<400> 102
   tgaggaatat tggtcaatgg gcgtgagcct gaaccagcca agccgcgtga gggaagaagg 60
   cgccaggcgt cgtaaacctc ttttgccggg gaacaaaggg cgccacgtgt ggcgttgtga 120
   gtgtacccgg agaaaaagca tcggctaact ccgtgcc 157
<210> 103
   <211> 157
   <212> DNA
   <213> Bacteroidales 16S rRNA gene V3 region
<400> 103
   tgaggaatat tggtcaatgg gcgtgagcct gaaccagcca agccgcgtga gggaggaagg 60
   cgccaggcgt cgtaaacctc ttttgccggg gaacaaaggg cgccacgtgt ggcgttgtga 120
   gtgtacccgg agaaaaagca tcggctaact ccgtgcc 157
<210> 104
   <211> 156
   <212> DNA
   <213> Bacteroidales 16S rRNA gene V3 region
<400> 104
   tgaggaatat tggtcaatgg gcgtgagcct gaaccagcca agccgcgtga gggaggaagg 60
   cgccaggcgt cgtaaacctc ttttgccggg gaacaaaggg cgccacgtgt ggcgttgtga 120
   gtgtacccgg agaaaagcat cggctaactc cgtgcc 156
<210> 105
   <211> 158
   <212> DNA
   <213> Bacteroidales 16S rRNA gene V3 region
<400> 105
   tgaggaatat tggtcaatgg gcgtgagcct gaaccagcca agccgcgtga gggaggaagg 60
   cgccaggcgt cgtaaacctc ttttgccggg gaacaaaggg cgccacgtgt ggcgttgtga 120
   gtgtacccgg agaaaaaagc atcggctaac tccgtgcc 158
<210> 106
   <211> 157
   <212> DNA
   <213> Butyricimonas 16S rRNA gene V3 region
<400> 106
   tgaggaatat tggtcaatgg gcgggagcct gaaccagcca agccgcgtga gggaataagg 60
   cgccaagcgt cgtaaacctc ttttgtcagg gaacaaaagc gggcacgcgt gcccgtccga 120
   gtgtacctga agaaaaagca tcggctaact ccgtgcc 157
<210> 107
   <211> 158
   <212> DNA
   <213> Porphyromonadaceae 16S rRNA gene V3 region
<400> 107
   tgaggaatat tggtcaatgg gcgagagcct gaaccagcca agccgcgtga aggaagaagg 60
   tgctaagcat tgtaaacttc ttttgtcagg gaacaaagag cgcgacgagt cgcgccgtga 120
   gtgtacctga agaaaaagac atcggctaac tccgtgcc 158
<210> 108
   <211> 157
   <212> DNA
   <213> Porphyromonadaceae 16S rRNA gene V3 region
<400> 108
   tgaggaatat tggtcaatgg gcgagagcct gaaccagcca agccgcgtga aggaagaagg 60
   tgctaagcat tgtaaacttc ttttgtcagg gaacaaagag cgcgacgagt cgcgccgtga 120
   gtgtacctga agaaaaagca tcggctaact ccgtgcc 157
<210> 109
   <211> 156
   <212> DNA
   <213> Porphyromonadaceae 16S rRNA gene V3 region
<400> 109
   tgaggaatat tggtcaatgg gcgagagcct gaaccagcca agccgcgtga aggaagaagg      60
   tgctaagcat tgtaaacttc ttttgtcagg gaacaaagag cgcgacgagt cgcgccgtga     120
   gtgtacctga agaaaagcat cggctaactc cgtgcc                               156
<210> 110
   <211> 157
   <212> DNA
   <213> Porphyromonadaceae 16S rRNA gene V3 region
<400> 110
   tgaggaatat tggtcaatgg tcggaagact gaaccagcca agccgcgtga aggaagaagg      60
   tgctcggcat cgtaaacttc ttttgtcagg gaacaaaggg cggtacgtgt accgctgtga     120
   gtgtacctga agaaaaagca tcggctaact ccgtgcc                              157
<210> 111
   <211> 158
   <212> DNA
   <213> Porphyromonadaceae 16S rRNA gene V3 region
<400> 111
   tgaggaatat tggtcaatgg tcggaagact gaaccagcca agccgcgtga aggaagaagg      60
   tgctcggcat cgtaaacttc ttttgtcagg gaacaaaggg cggtacgtgt accgctgtga     120
   gtgtacctga agaaaaaagc atcggctaac tccgtgcc                             158
<210> 112
   <211> 157
   <212> DNA
   <213> Porphyromonadaceae 16S rRNA gene V3 region
<400> 112
   tgaggaatat tggtcaatgg gcgtgagcct gaaccagcca agccgcgtga aggaagaagg      60
   tgcagggcat cgtaaacttc ttttgccggg gaacaataag cgggactagt cccgcgacga     120
   gtgtacccgg agaaaaagca tcggctaact ccgtgcc                              157
<210> 113
   <211> 157
   <212> DNA
   <213> Porphyromonadaceae 16S rRNA gene V3 region
<400> 113
   tgaggaatat tggtcaatgg gcgtgagcct gaaccagcca agccgcgtga aggaagaagg 60
   tgcagggcat cgtaaacttc ttttgccggg gaacaataag cgggactagt cccgcgacga 120
   gtgtacccgg agaaaagaca tcggctaact ccgtgcc 157
<210> 114
   <211> 157
   <212> DNA
   <213> Porphyromonadaceae 16S rRNA gene V3 region
<400> 114
   tgaggaatat tggtcaatgg tcgggagact gaaccagcca agccgcgtga gggatggagg 60
   tacagagtat cgtaaacctc ttttgtcagg gaacaaaggg cgccacgtgt ggcgctatga 120
   gggtacctga agaaaaagca tcggctaact ccgtgcc 157
<210> 115
   <211> 157
   <212> DNA
   <213> Barnesiella 16S rRNA gene V3 region
<400> 115
   tgaggaatat tggtcaatgg gagagatcct gaaccagcca agccgcgtga gggaagacgg 60
   cactacgtgt tgtaaacctc ttttgccggg gaacaaaagc ggggacgcgt ccccgtccgc 120
   gtgtacccgg agaaaaagca tcggctaact ccgtgcc 157
<210> 116
   <211> 157
   <212> DNA
   <213> Barnesiella 16S rRNA gene V3 region
<400> 116
   tgaggaatat tggtcaatgg gcgggagcct gaaccagcca agtcgcgtga gggaagacgg 60
   ccctacgggt tgtaaacctc ttttgtcggg gagcaaagag cgccacgcgt ggcgagatga 120
   gagtacccga agaaaaagca tcggctaact ccgtgcc 157
<210> 117
   <211> 158
   <212> DNA
   <213> Barnesiella 16S rRNA gene V3 region
<400> 117
   tgaggaatat tggtcaatgg gcgggagcct gaaccagcca agtcgcgtga gggaagacgg 60
   ccctacgggt tgtaaacctc ttttgtcggg gagcaaagag cgccacgcgt ggcgagatga 120
   gagtacccga agaaaaagac atcggctaac tccgtgcc 158
<210> 118
   <211> 157
   <212> DNA
   <213> Porphyromonadaceae 16S rRNA gene V3 region
<4> 118
<210> 119
   <211> 157
   <212> DNA
   <213> Bacteroidales 16S rRNA gene V3 region
<400> 119
   tgaggaatat tggtcaatgg gcgggagcct gaaccagcca agccgcgtga gggaataagg 60
   ccctacgggt cgtaaacctc ttttgtcggg gaacaaaacc ggagacgagt ctccggctgc 120
   gtgtacccga agaaaaagca tcggctaact ccgtgcc 157
<210> 120
   <211> 158
   <212> DNA
   <213> Bacteroidales 16S rRNA gene V3 region
<400> 120
   tgaggaatat tggtcaatgg gcgggagcct gaaccagcca agccgcgtga gggaagaagg 60
   cgctcagcgt cgtaaacctc tttagccggg gaacaaagag ctgctcggga agcagcgttg 120
   agcgtacccg gagaataagc atcggctaac tccgtgcc 158
<210> 121
   <211> 158
   <212> DNA
   <213> Porphyromonadaceae 16S rRNA gene V3 region
<400> 121
   tgaggaatat tggtcaatgg gcggaagcct gaaccagcca agtcgcgtga gggacgacgg 60
   tcctacggat tgtaaacctc ttttgccggg gagcaatgcg cggtacgcgt accgcgacgg 120
   agagtacccg gagaaaaagc atcggctaac tccgtgcc 158
<210> 122
   <211> 157
   <212> DNA
   <213> Porphyromonadaceae 16S rRNA gene V3 region
<400> 122
   tgaggaatat tggtcaatgg gcgagagcct gaaccagcca agtcgcgtga gggaagacgg 60
   ccctacgggt tgtaaacctc ttttgtcggg gagcaaagag cgccacgcgt ggcgagatga 120
   gagtacccga agaaaagaca tcggctaact ccgtgcc 157
<210> 123
   <211> 157
   <212> DNA
   <213> Barnesiella 16S rRNA gene V3 region
<400> 123
   tgaggaatat tggtcaatgg gcgcgagcct gaaccagcca agtcgcgtga gggaagacgg 60
   ccctacgggt tgtaaacctc ttttgtcggg gagcaaggac tgccacgagt ggcagggcga 120
   gagtacccga agaaaaagca tcggctaact ccgtgcc 157
<210> 124
   <211> 157
   <212> DNA
   <213> Porphyromonadaceae 16S rRNA gene V3 region
<400> 124
   tgaggaatat tggtcaatgg gcgggagcct gaaccagcca agtcgcgtga gggaagacgg 60
   tcttacggat tgtaaacctc ttttgccggg gagcaaaggg cgccacgcgt ggcgtttcga 120
   gagtacccgg agaaaaagca tcggctaact ccgtgcc 157
<210> 125
   <211> 157
   <212> DNA
   <213> Porphyromonadaceae 16S rRNA gene V3 region
<400> 125
   tgaggaatat tggtcaatgg ccgagaggct gaaccagcca agtcgcgtga gggaagacgg 60
   tcctatggat tgtaaacctc ttttgtcggg gagcaaaagg cgtcacgtgt gacgctatga 120
   gagtacccga agaaaaagca tcggctaact ccgtgcc 157
<210> 126
   <211> 157
   <212> DNA
   <213> Bacteroidales 16S rRNA gene V3 region
<400> 126
   tgaggaatat tggtcaatgg ccggaaggct gaaccagcca agccgcgtga gggaggaagg 60
   cgcagagcgt cgcagacctc ttttgccggg ggacaaaagg ccggactcgt ccggtcctga 120
   gggtacccgg agaaaaagca tcggctaact ccgtgcc 157
<210> 127
   <211> 158
   <212> DNA
   <213> Barnesiella 16S rRNA gene V3 region
<400> 127
   tgaggaatat tggtcaatgg gcgcgagcct gaaccagcca agtcgcgtga gggatgacgg 60
   ccctacgggt tgtaaacctc ttttgtcggg gagcaaattc cgttacgtgt aacggagtcg 120
   agagtacccg aagaaaaagc atcggctaac tccgtgcc 158
<210> 128
   <211> 158
   <212> DNA
   <213> Porphyromonadaceae 16S rRNA gene V3 region
<400> 128
   tgaggaatat tggtcaatgg gcggaagcct gaaccagcca agtcgcgtga gggaagacgg 60
   tcctatggat tgtaaacctc ttttgcaggg gagcaaggca cggtacgtgt accgtgaagg 120
   agagtaccct gagaaaaagc atcggctaac tccgtgcc 158
<210> 129
   <211> 158
   <212> DNA
   <213> Porphyromonadaceae 16S rRNA gene V3 region
<400> 129
   tgaggaatat tggtcaatgg gcggaagcct gaaccagcca agtcgcgtga gggaagacgg 60
   tcctatggat tgtaaacctc ttttgtcggg gagcaaagcc gctcacgtgt gagcggaagg 120
   agagtacccg aagaaaaagc atcggctaac tccgtgcc 158
<210> 130
   <211> 158
   <212> DNA
   <213> Porphyromonadaceae 16S rRNA gene V3 region
<400> 130
   tgaggaatat tggtcaatgg gcggaagcct gaaccagcca agtcgcgtga gggaggacgg 60
   tcctatggat tgtaaacctc ttttgtcggg gagcaaagcc gctcacgtgt gagcggaagg 120
   agagtacccg aagaaaaagc atcggctaac tccgtgcc 158
<210> 131
   <211> 157
   <212> DNA
   <213> Barnesiella 16S rRNA gene V3 region
<400> 131
   tgaggaatat tggtcaatgg ccgaagggct gaaccagcca agtcgcgtga gggaagacgg 60
   ccctacgggt tgtaaacctc ttttgccggg gagcaaaggc ggtcactggt gaccggatga 120
   gagtacccgg agaaaaagca tcggctaact ccgtgcc 157
<210> 132
   <211> 158
   <212> DNA
   <213> Porphyromonadaceae 16S rRNA gene V3 region
<400> 132
   tgaggaatat tggtcaatgg gcgagagcct gaaccagcca agtcgcgtga gggaagacgg 60
   tcctatggat tgtaaacctc ttttgtcagg gagcaaggag ggccacgagt ggcgcttcgg 120
   agagtacctg aagaaaaagc atcggctaac tccgtgcc 158
<210> 133
   <211> 158
   <212> DNA
   <213> Barnesiella 16S rRNA gene V3 region
<400> 133
   tgaggaatat tggtcaatgg ccgagaggct gaaccagcca agtcgcgtga gggaagacgg 60
   ccctacgggt tgtaaacctc ttttgtcggg gagcaaacag cgcaacgcgc ttgcgcattg 120
   agagtacccg aagaaaaagc atcggctaac tccgtgcc 158
<210> 134
   <211> 157
   <212> DNA
   <213> Barnesiella 16S rRNA gene V3 region
<400> 134
   tgaggaatat tggtcaatgg gcgagagcct gaaccagcca agtcgcgtga gggaagacgg 60
   ccctacgggt tgtaaacctc ttttgtcggg gagcaaagag cgccacgcgt ggcgagatga 120
   gagtacccga agaaaaagca tcggctaact ccgtgcc 157
<210> 135
   <211> 157
   <212> DNA
   <213> Porphyromonadaceae 16S rRNA gene V3 region
<400> 135
   tgaggaatat tggtcaatgg gcgagagcct gaaccagcca agtcgcgtga gggaagacgg 60
   ccctatgggt tgtaaacctc ttttgccggg gagcaaagaa ccgcacgtgt gcggtctgga 120
   gagtacccgg agaaaaagca tcggctaact ccgtgcc 157
<210> 136
   <211> 158
   <212> DNA
   <213> Porphyromonadaceae 16S rRNA gene V3 region
<400> 136
   tgaggaatat tggtcaatgg gcggaagcct gaaccagcca agtcgcgtga gggaataagg 60
   ccctaagggt cgtaaacctc ttttgccggg gagcaatggt tcgcttgcga gcggacaggg 120
   agagtacccg gagaaaaagc atcggctaac tccgtgcc 158
<210> 137
   <211> 157
   <212> DNA
   <213> Barnesiella 16S rRNA gene V3 region
<400> 137
   tgaggaatat tggtcaatgg ccgtaaggct gaaccagcca agtcgcgtga gggaagacgg 60
   ccctacgggt tgtaaacctc ttttgccggg gagcaaaagg cgccacgcgt ggcgtttcga 120
   gagtacccgg agaaaaagca tcggctaact ccgtgcc 157
<210> 138
   <211> 158
   <212> DNA
   <213> Barnesiella 16S rRNA gene V3 region
<400> 138
   tgaggaatat tggtcaatgg gcgtaagcct gaaccagcca agtcgcgtga gggaagacgg 60
   ccctatgggt tgtaaacctc ttttgtcggg gagcaaagcc gcccacgtgt gggcggaagg 120
   agagtacccg aagaaaaagc atcggctaac tccgtgcc 158
<210> 139
   <211> 158
   <212> DNA
   <213> Barnesiella 16S rRNA gene V3 region
<400> 139
   tgaggaatat tggtcaatgg gcgtaagcct gaaccagcca agtcgcgtga gggaagacgg 60
   ccctatgggt tgtaaacctc ttttgtcggg gagcaaagcc gcccacgagt gggcggaagg 120
   agagtacccg aagaaaaagc atcggctaac tccgtgcc 158
<210> 140
   <211> 159
   <212> DNA
   <213> Porphyromonadaceae 16S rRNA gene V3 region
<400> 140
   tgaggaatat tggtcaatgg gcgtaagcct gaaccagcca agtcgcgtga gggaagacgg 60
   ccctatgggt tgtaaacctc ttttgtcggg gagcaaagcc gcccacgagt gggcggaagg 120
   agagtacccg aagaaaaaga catcggctaa ctccgtgcc 159
<210> 141
   <211> 158
   <212> DNA
   <213> Porphyromonadaceae 16S rRNA gene V3 region
<400> 141
   tgaggaatat tggtcaatgg gcgtaagcct gaaccagcca agtcgcgtga gggaagacgg 60
   ccctatgggt tgtaaacctc ttttgtcggg gagcaaagcc gcccacgagt gggcggaagg 120
   agagtacccg aagaaaagac atcggctaac tccgtgcc 158
<210> 142
   <211> 158
   <212> DNA
   <213> Porphyromonadaceae 16S rRNA gene V3 region
<400> 142
   tgaggaatat tggtcaatgg gcggtagcct gaaccagcca agtcgcgtga gggaagacgg 60
   tcctatggat tgtaaacctc ttttgccggg gagcaaggcc atgtacgtgt acgtggcctg 120
   agagtacccg gagaaaaagc atcggctaac tccgtgcc 158
<210> 143
   <211> 158
   <212> DNA
   <213> porphyromonadaceae 16S rRNA gene V3 region
<400> 143
   tgaggaatat tggtcaatgg gcgcgagcct gaaccagcca agtcgcgtga gggaagacgg 60
   tcctaaggat tgtaaacctc ttttgtcagg gagcaaggag cgccacgtgt ggcgcggcga 120
   gagtacctga agaaaaagac atcggctaac tccgtgcc 158
<210> 144
   <211> 157
   <212> DNA
   <213> Porphyromonadaceae 16S rRNA gene V3 region
<400> 144
   tgaggaatat tggtcaatgg gcgcgagcct gaaccagcca agtcgcgtga gggaagacgg 60
   tcctaaggat tgtaaacctc ttttgtcagg gagcaaggag cgccacgtgt ggcgcggcga 120
   gagtacctga agaaaaagca tcggctaact ccgtgcc 157
<210> 145
   <211> 157
   <212> DNA
   <213> Porphyromonadaceae 16S rRNA gene V3 region
<400> 145
   tgaggaatat tggtcaatgg gcgagagcct gaaccagcca agtcgcgtga gggaagacag 60
   tcctatggat tgtaaacctc ttttgccggg gagcaaagag cggcacgtgt gccgcgccga 120
   gagtacccgg agaaaaagca tcggctaact ccgtgcc 157
<210> 146
   <211> 157
   <212> DNA
   <213> Butyricimonas 16S rRNA gene V3 region
<400> 146
   tgaggaatat tggtcaatgg gcgagagcct gaaccagcca agtcgcgtga gggaagacgg 60
   tcctatggat tgtaaacctc ttttgccggg gagcaaagag cggcacgtgt gccgcgccga 120
   gagtacccgg agaaaaagca tcggctaact ccgtgcc 157
<210> 147
   <211> 157
   <212> DNA
   <213> Porphyromonadaceae 16S rRNA gene V3 region
<400> 147
   tgaggaatat tggtcaatgg gcgagagcct gaaccagcca agtcgcgtga gggaagacgg 60
   tcctatggat tgtaaacctc ttttgtcggg gagcaacgaa ggcacgtgtg ccagaagcga 120
   gattacccga agaaaaagca tcggctaact ccgtgcc 157
<210> 148
   <211> 158
   <212> DNA
   <213> Porphyromonadaceae 16S rRNA gene V3 region
<400> 148
   tgaggaatat tggtcaatgg gcgagagcct gaaccagcca agtcgcgtga gggaagacgg 60
   tcctatggat tgtaaacctc ttttgtcggg gagcaacgaa ggcacgtgtg ccagaagcga 120
   gattacccga agaaaaagac atcggctaac tccgtgcc 158
<210> 149
   <211> 157
   <212> DNA
   <213> Porphyromonadaceae 16S rRNA gene V3 region
<400> 149
   tgaggaatat tggtcaatgg gcgagagcct gaaccagcca agtcgcgtga gggaagacgg 60
   tcctatggat tgtaaacctc ttttgtcggg gagcaacgaa ggcacgtgtg cctgaagcga 120
   gattacccga agaaaaagca tcggctaact ccgtgcc 157
<210> 150
   <211> 158
   <212> DNA
   <213> Porphyromonadaceae 16S rRNA gene V3 region
<400> 150
   tgaggaatat tggtcaatgg gcgggagcct gaaccagcca agtcgcgtga gggaagacgg 60
   tcctatggat tgtaaacctc ttttgtcggg gagcaaagag gccacgtgtg gtcaaaagcg 120
   agagtacccg aagaaaaagc atcggctaac tccgtgcc 158
<210> 151
   <211> 159
   <212> DNA
   <213> Porphyromonadaceae 16S rRNA gene V3 region
<400> 151
   tgaggaatat tggtcaatgg gcgggagcct gaaccagcca agtcgcgtga gggaagacgg 60
   tcctatggat tgtaaacctc ttttgtcggg gagcaaagag gccacgtgtg gtcaaaagcg 120
   agagtacccg aagaaaaaga catcggctaa ctccgtgcc 159
<210> 152
   <211> 157
   <212> DNA
   <213> Porphyromonadaceae 16S rRNA gene V3 region
<400> 152
   tgaggaatat tggtcaatgg gcggaagcct gaaccagcca agtcgcgtga gggaagacgg 60
   tcctatggat tgtaaacctc ttttgtcggg gagcaaggat ccgcacgagt gcggaggcga 120
   gagtacccga agaaaaagca tcggctaact ccgtgcc 157
<210> 153
   <211> 158
   <212> DNA
   <213> Porphyromonadaceae 16S rRNA gene V3 region
<400> 153
   tgaggaatat tggtcaatgg gcggaagcct gaaccagcca agtcgcgtga gggaagacgg 60
   tcctatggat tgtaaacctc ttttgtcggg gagcaaggat ccgcacgagt gcggaggcga 120
   gagtacccga agaaaaagac atcggctaac tccgtgcc 158
<210> 154
   <211> 157
   <212> DNA
   <213> Butyricimonas 16S rRNA gene V3 region
<400> 154
   tgaggaatat tggtcaatgg gcgagagcct gaaccagcca agtcgcgtga gggaagaatg 60
   gtctatggcc tgtaaacctc ttttgtcagg gaagaataag gatgacgagt cattcgatgc 120
   cagtacttga cgaataagca tcggctaact ccgtgcc 157
<210> 155
   <211> 157
   <212> DNA
   <213> Alistipes 16S rRNA gene V3 region
<400> 155
   tgaggaatat tggtcaatgg acgcaagtct gaaccagcca tgccgcgtgc aggaagacgg 60
   ctctatgagt tgtaaactgc ttttgtacga gggtaaaccc ggatacgtgt atccggctga 120
   aagtatcgta cgaataagga tcggctaact ccgtgcc 157
<210> 156
   <211> 162
   <212> DNA
   <213> unknown bacterium 16S rRNA gene V3 region
<400> 156
   ctaaggatat tccgcaacgg gcggaagccc ggcggagcga cgccgcgtgg acgaggaagg 60
   ccggaaggtt gcagagtcct tttgcggggg aagaaggagc cgcggaggga atgccgcggc 120
   ggcgaccgaa ccccgcgaat aaggggcggc taattacgtg cc 162
<210> 157
   <211> 155
   <212> DNA
   <213> Bifidobacterium 16S rRNA gene V3 region
<400> 157
   tggggaatat tgcacaatgg gcgcaagcct gatgcagcga cgccgcgtgc gggatggagg      60
   ccttcgggtt gtaaaccgct tttgttcaag ggcaaggcac ggcttcgggc cgtgttgagt     120
   ggattgttcg aataagcacc ggctaactac gtgcc                                155
<210> 158
   <211> 137
   <212> DNA
   <213> Lachnospiraceae 16S rRNA gene V3 region
<400> 158
   tggggaatat tgcacaatgg ggggaaccct gatgcagcga cgccgcgtgg gtgaagaagt      60
   atttcggtat gtaaagccct atcagcaggg aagaaaaaga cggtacctga ctaagaagcc     120
   ccggctaact acgtgcc                                                    137
<210> 159
   <211> 137
   <212> DNA
   <213> Hespellia 16S rRNA gene V3 region
<400> 159
   tggggaatat tgcacaatgg gggaaaccct gatgcagcga cgccgcgtga aggaagaagt      60
   atttcggtat gtaaacttct atcagcaggg aagaaaatga cggtatctga ctaagaagcc     120
   ccggctaact acgtgcc                                                    137
<210> 160
   <211> 137
   <212> DNA
   <213> Marvinbryantia 16S rRNA gene V3 region
<400> 160
   tgggggatat tgcacaatgg gggaaaccct gatgcagcga cgccgcgtgg gtgaagaagt      60
   atttcggtat gtaaagccct atcagcaggg aagaaaatga cagtacctga ataagaagcc     120
   ccggctaact acgtgcc                                                    137
<210> 161
   <211> 138
   <212> DNA
   <213> Lachnospiraceae 16S rRNA gene V3 region
<400> 161
   tgggggatat tgcacaatgg ggggaaccct gatgcagcga cgccgcgtgg gtgaagaagt 60
   atttcggtat gtaaagccct atcagcaggg aagaaagaag acggtacctg agtaagaagc 120
   cccggctaac tacgtgcc 138
<210> 162
   <211> 139
   <212> DNA
   <213> Lachnospiraceae 16S rRNA gene V3 region
<400> 162
   tggggagtat tgcacaatgg gggaaacccg tgatgcagcg acgccgcgtg agtgaagaag 60
   tatttcggta tgtaaagctc tatcagcagg gaagaaaata gacggtacct gactaagaag 120
   ccccggctaa ctacgtgcc 139
<210> 163
   <211> 140
   <212> DNA
   <213> Lachnospiraceae 16S rRNA gene V3 region
<400> 163
   tgggggatat tggacaatgg ggggaaccct gatccagcga cgccgcgtga gtgaagaagt 60
   atttcggtat gtaaagctct atcagcaggg aagaaagaaa tgacggtacc tgaccaagaa 120
   gccccggcta actacgtgcc 140
<210> 164
   <211> 142
   <212> DNA
   <213> Lachnospiraceae 16S rRNA gene V3 region
<400> 164
   tggggaatat tgcacaatgg gggaaaccct gatgcagcga cgccgcgtga gtgaagaagt 60
   aattcgttac gtaaagctct atcagcaggg aagaaaaaga aatgacggta cctgattaag 120
   aagccccggc taactacgtg cc 142
<210> 165
   <211> 138
   <212> DNA
   <213> Lachnospiraceae 16S rRNA gene V3 region
<400> 165
   tggggaatat tgcacaatgg ggggaaccct gatgcagcaa tgccgcgtga gtgaagaagt 60
   atttcggtat gtaaagctct atcagcaggg aagaaaaaag acggtacctg actaagaagc 120
   cccggctaac tacgtgcc 138
<210> 166
   <211> 138
   <212> DNA
   <213> Lachnospiraceae 16S rRNA gene V3 region
<400> 166
   tggggaatat tgcacaatgg gggaaaccct gatgcagcaa tgccgcgtga gtgaagaagt 60
   atttcggtat gtaaagctct atcagcaggg aagaaaaaag acggtacctg actaagaagc 120
   cccggctaac tacgtgcc 138
<210> 167
   <211> 139
   <212> DNA
   <213> Lachnospiraceae 16S rRNA gene V3 region
<400> 167
   tggggaatat tgcacaatgg aggaaactct gatgcagcga cgccgcgtga gtgaagaagt 60
   aattcgttac gtaaagctct atcagcaggg aagaaaaaat gacggtacct gactaagaag 120
   caccggctaa atacgtgcc 139
<210> 168
   <211> 139
   <212> DNA
   <213> Lachnospiraceae 16S rRNA gene V3 region
<400> 168
   tggggaatat tgcacaatgg aggaaactct gatgcagcga cgccgcgtga gtgaagaagt 60
   aattcgttac gtaaagctct atcagcaggg agaaaaaaat gacggtacct gactaagaag 120
   caccggctaa atacgtgcc 139
<210> 169
   <211> 137
   <212> DNA
   <213> Oribacterium 16S rRNA gene V3 region
<400> 169
   tgggggatat tgcacaatgg gggaaaccct gatgcagcga cgccgcgtga gtgaagaagt 60
   atttcggtat gtaaagctct atcagcaggg aagaagatga cagtacctga ctaagaagcc 120
   ccggctaact acgtgcc 137
<210> 170
   <211> 137
   <212> DNA
   <213> Coprococcus 16S rRNA gene V3 region
<400> 170
   tggggaatat tgcacaatgg ggggaaccct gatgcagcaa cgccgcgtga gtgaagaagt 60
   atttcggtat gtaaagctct atcagcaggg aagaaaatga cggtacctga ctaagaagct 120
   ccggctaaat acgtgcc 137
<210> 171
   <211> 137
   <212> DNA
   <213> Ruminococcus 16S rRNA gene V3 region
<400> 171
   tggggaatat tgcacaatgg gggaaaccct gatgcagcga tgccgcgtgg aggaagaagg 60
   ttttcggatt gtaaactcct gtcttaaagg acgataatga cggtacttta ggaggaagct 120
   ccggctaact acgtgcc 137
<210> 172
   <211> 138
   <212> DNA
   <213> Roseburia 16S rRNA gene V3 region
<400> 172
   tggggaatat tgcacaatgg gcgcaagcct gatgcagcga cgccgcgtga gtgaggaagt 60
   atttcggtat gtaaagctct atcagcaggg aagaagaatg acggtacctg actaagaagc 120
   accggctaaa tacgtgcc 138
<210> 173
   <211> 137
   <212> DNA
   <213> Lachnospiraceae 16S rRNA gene V3 region
<400> 173
   tggggaatat tgcacaatgg agggaactct gatgcagcga cgccgcgtga gtgaagaagt 60
   aattcgttat gtaaagctct gtcagcaggg aagaaagtga cggtacctga aaaagaagct 120
   ccggctaaat acgtgcc 137
<210> 174
   <211> 137
   <212> DNA
   <213> Lachnospiraceae 16S rRNA gene V3 region
<400> 174
   tggggaatat tgcacaatgg gggaaaccct gatgcagcga cgccgcgtga gtgaagaaat 60
   atttcggtat gtaaagctct atcagcaggg aagaaaatga cggtacctga gtaagaagcc 120
   ccggctaact acgtgcc 137
<210> 175
   <211> 138
   <212> DNA
   <213> Lachnospiraceae 16S rRNA gene V3 region
<400> 175
   tggggaatat tgcacaatgg gggaaaccct gatgcagcga cgccgcgtga gtgaagaagt 60
   aattcgttat gtaaagctct atcagcaagg aagaaaaaag acggtacttg actaagaagc 120
   cccggctaaa tacgtgcc 138
<210> 176
   <211> 137
   <212> DNA
   <213> Lachnospiraceae 16S rRNA gene V3 region
<400> 176
   tgggggatat tgcacaatgg gggaaaccct gatgcagcga cgccgcgtgg gtgaagaagt 60
   atttcggtat gtaaagccct atcagcaggg aagaagatga cagtacctga ctaagaagcc 120
   ccggctaact acgtgcc 137
<210> 177
   <211> 137
   <212> DNA
   <213> Lachnospiraceae 16S rRNA gene V3 region
<400> 177
   tgggggatat tgcacaatgg ggggaaccct gatgcagcga cgccgcgtgg gtgaaggagt 60
   actccggtat gtaaagccct atcggcaggg aagaagatga cggtacctga ctaagaagct 120
   ccggctaaat acgtgcc 137
<210> 178
   <211> 137
   <212> DNA
   <213> Butyrivibrio 16S rRNA gene V3 region
<400> 178
   tgggggatat tgcacaatgg gggaaaccct gatgcagcga cgccgcgtga gtgaagaagt 60
   atttcggtat gtaaagctct atcagcaggg aagaaagtga cagtacctga gtaagaagcc 120
   ccggctaact acgtgcc 137
<210> 179
   <211> 140
   <212> DNA
   <213> Lachnospiraceae 16S rRNA gene V3 region
<400> 179
   tgggggatat tggacaatgg ggggaaccct gatccagcga cgccgcgtga gtgaagaagt 60
   atctcggtat gtaaagctct atcagcaggg aagaaagaaa tgacggtacc tgagtaagaa 120
   gccccggcta actacgtgcc 140
<210> 180
   <211> 140
   <212> DNA
   <213> Lachnospiraceae 16S rRNA gene V3 region
<400> 180
   tgggggatat tggacaatgg ggggaaccct gatccagcga cgccgcgtga gtgaagaagt 60
   atctcggtat gtaaagctct gtcagcaggg aagaaagaaa tgacggtacc tgaccaagaa 120
   gccccggcta actacgtgcc 140
<210> 181
   <211> 140
   <212> DNA
   <213> lachnospiraceae 16S rRNA gene V3 region
<400> 181
   tgggggatat tggacaatgg ggggaaccct gatccagcga cgccgcgtga gtgaagaagt 60
   atttcggtat gtaaagctct gtcagcaggg aagaaagaaa tgacggtacc tgaagaagaa 120
   gccccggcta actacgtgcc 140
<210> 182
   <211> 138
   <212> DNA
   <213> Lachnospiraceae 16S rRNA gene V3 region
<400> 182
   tgggggatat tgcacaatgg ggggaaccct gatgcagcga cgccgcgtgg gtgaaggagt 60
   gcttcggcat gtaaagccct atcggcaggg aagaagaagg acggtacctg actaagaagc 120
   cccggctaac tacgtgcc 138
<210> 183
   <211> 137
   <212> DNA
   <213> Lachnospiraceae 16S rRNA gene V3 region
<400> 183
   tgggggatat tgcacaatgg ggggaaccct gatgcagcga cgccgcgtgg gtgaagaagc 60
   gccccggcgc gtaaagccct atcggcaggg aagaagatga cggtacctgg ctaagaagcc 120
   ccggctaact acgtgcc 137
<210> 184
   <211> 135
   <212> DNA
   <213> Lachnospiraceae 16S rRNA gene V3 region
<400> 184
   ggggatattg cacaatgggg gaacccgtga tgcagcgacg ccgcgtgggt gaagaagcgc 60
   cccggcgcgt aaagccctat cggcagggaa gaagatgacg gtacctggct aagaagcccc 120
   ggctaactac gtgcc 135
<210> 185
   <211> 137
   <212> DNA
   <213> Lachnospiraceae 16S rRNA gene V3 region
<400> 185
   tggggaatat tgcacaatgg ggggaaccct gatgcagcga cgccgcgtga gtgaagaagt 60
   atttcggtat gtaaagctct atcagcagga acgaagaaga cggtacctga ctaagaagcc 120
   ccggctaact acgtgcc 137
<210> 186
   <211> 138
   <212> DNA
   <213> Lachnospiraceae 16S rRNA gene V3 region
<400> 186
   tggggaatat tgcacaatgg gggaaaccct gatgcagcga cgccgcgtga gtgaagaagt 60
   aattcgttac gtaaagctct atcagcagga aagaaagaag acggtacctg actaagaagc 120
   cccggctaac tacgtgcc 138
<210> 187
   <211> 137
   <212> DNA
   <213> Roseburia 16S rRNA gene V3 region
<400> 187
   tgggggatat tgcacaatgg gggaaaccct gatgcagcga cgccgcgtga gcgaagaagt 60
   atttcggtat gtaaagctct atcagcgggg aagagaatga cggtacccga ctaagaagct 120
   ccggctaaat acgtgcc 137
<210> 188
   <211> 137
   <212> DNA
   <213> Lachnospiraceae 16S rRNA gene V3 region
<400> 188
   tgggggatat tgcacaatgg gggaaaccct gatgcagcga cgccgcgtga gtgaagaagt 60
   atttcggtat gtaaagctct atcagcaggg aagaagatga cagtacctga ataagaagcc 120
   ccggctaact acgtgcc 137
<210> 189
   <211> 137
   <212> DNA
   <213> Coprococcus 16S rRNA gene V3 region
<400> 189
   tggggaatat tgcacaatgg gggaaaccct gatgcagcga cgccgcgtga gcgaggaagt 60
   atttcggtat gtaaagctct atcagcaggg aagaaaatga cggtacctga ctaagaagcc 120
   ccggctaact acgtgcc 137
<210> 190
   <211> 138
   <212> DNA
   <213> unknown bacterium 16S rRNA gene V3 region
<400> 190
   tggggaatat tgggcaatgg gcgcaagcct gacccagcaa cgccgcgtga gcgatgaagg 60
   tcttcggatt gtaaagctct gtcgcagggg acgaagtatg acggtaccct gtaagaaagc 120
   cccggcaaac tacgtgcc 138
<210> 191
   <211> 138
   <212> DNA
   <213> Lachnospiraceae 16S rRNA gene V3 region
<400> 191
   tggggaatat tgcacaatgg gggaaaccct gatgcagcga cgccgcgtga gcgaagaagt 60
   atctcggtat gtaaagctct atcagcaggg aagaagaatg acggtacctg agtaagaagc 120
   accggctaaa tacgtgcc 138
<210> 192
   <211> 137
   <212> DNA
   <213> Lachnospiraceae 16S rRNA gene V3 region
<400> 192
   tgggggatat tgcacaatgg gggaaaccct gatgcagcga cgccgcgtga gcgaagaagt 60
   atttcggtat gtaaagctct atcagcaggg aagaaaatga cggtacctga acaagaagct 120
   ccggctaaat acgtgcc 137
<210> 193
   <211> 137
   <212> DNA
   <213> Coprococcus 16S rRNA gene V3 region
<400> 193
   tggggaatat tgcacaatgg ggggaaccct gatgcagcaa cgccgcgtga gtgaagaagt 60
   atttcggtat gtaaagctct atcagcaggg aagaaaatga cggtacctga ctaagaagca 120
   ccggctaaat acgtgcc 137
<210> 194
   <211> 138
   <212> DNA
   <213> Lachnospiraceae 16S rRNA gene V3 region
<400> 194
   tgggggatat tgcacaatgg ggggaaccct gatgcagcga cgccgcgtga gtgaaggagt 60
   acttcggtat gtaaagctct atcagcaggg aagaagcaag acggtacctg accaagaagc 120
   cccggctaac tacgtgcc 138
<210> 195
   <211> 137
   <212> DNA
   <213> Lachnospiraceae 16S rRNA gene V3 region
<400> 195
   tggggaatat tgcacaatgg ggggaaccct gatgcagcga cgccgcgtga gcgaagaagt 60
   atttcggtat gtaaagctct atcagcaggg aagaaaatga cggtacctga ctaagaagcc 120
   ccggctaact acgtgcc 137
<210> 196
   <211> 140
   <212> DNA
   <213> unknown bacterium 16S rRNA gene V3 region
<400> 196
   tggggaatat tgggcaatgg aggcaactct gacccagcaa cgccgcgtga gcgatgaagg 60
   tcttcggatt gtaaagctct ttaagtgggg acgaagaaag tgactgtacc cacagaataa 120
   gcctcggcta actacgtgcc 140
<210> 197
   <211> 140
   <212> DNA
   <213> unknown bacterium 16S rRNA gene V3 region
<400> 197
   tggggaatat tgggcaatgg aggaaactct gacccagcaa cgccgcgtga atgatgaagg 60
   tcttcggatt gtaaagttct tttctaaggg aagaagaaag tgacggtacc ttaggaataa 120
   gcctcggcta actacgtgcc 140
<210> 198
   <211> 137
   <212> DNA
   <213> marvinbryantia 16S rRNA gene V3 region
<400> 198
   tggggaatat tgcacaatgg ggggaaccct gatgcagcaa tgccgcgtgg gtgaagaagt 60
   accccggtat gtaaagccct atcagcaggg aagaaaatga cggtacctgg ctaagaagcc 120
   ccggctaact acgtgcc 137
<210> 199
   <211> 137
   <212> DNA
   <213> Lachnospiraceae 16S rRNA gene V3 region
<400> 199
   tggggaatat tgcacaatgg gggaaaccct gatgcagcga cgccgcgtga gcgaagaagt 60
   atttcggtat gtaaagctct atcagcaagg aagataatga cggtacttga ctaagaagct 120
   ccggctaaat acgtgcc 137
<210> 200
   <211> 140
   <212> DNA
   <213> Lachnospiraceae 16S rRNA gene V3 region
<400> 200
   tggggaatat tgcacaatgg gcgaaagcct gatgcagcaa cgccgcgtga aggaagaagg 60
   gtttcggctc gtaaacttct atcaacaggg acgaaggaag tgacggtacc tgaataagaa 120
   gccccggcta actacgtgcc 140
<210> 201
   <211> 137
   <212> DNA
   <213> Coprococcus 16S rRNA gene V3 region
<400> 201
   tggggaatat tgcacaatgg gggaaaccct gatgcagcaa cgccgcgtga gtgaagaagt 60
   atttcggtat gtaaagctct atcagcaggg aagaaaatga cggtacctga ctaagaagct 120
   ccggctaaat acgtgcc 137
<210> 202
   <211> 137
   <212> DNA
   <213> Ruminococcaceae 16S rRNA gene V3 region
<400> 202
   tggggaatat tgcacaatgg gggaaaccct gatgcagcga cgccgcgtgg aggaagaagg 60
   ccctcgggtt gtaaactcct gtctttgggg acgataatga cggtacccaa ggaggaagcc 120
   acggctaact acgtgcc 137
<210> 203
   <211> 137
   <212> DNA
   <213> Clostridiales 16S rRNA gene V3 region
<400> 203
   tggggaatat tgcacaatgg gggaaaccct gatgcagcga cgccgcgtgg aggaagaagg 60
   ccctcgggtt gtaaactcct gtcttcgggg acgataatga cggtacccga ggaggaagcc 120
   acggctaact acgtgcc 137
<210> 204
   <211> 137
   <212> DNA
   <213> Clostridiales 16S rRNA gene V3 region
<400> 204
   tgggggatat tgcacaatgg ggggaaccct gatgcagcga cgccgcgtga gcgaagaaga 60
   tcttcggatt gtaaagctct gtcttagggg acgatgatga cggtaccctg agaggaagcc 120
   acggctaact acgtgcc 137
<210> 205
   <211> 138
   <212> DNA
   <213> Ruminococcaceae 16S rRNA gene V3 region
<400> 205
   tgggggatat tgcgcaatgg gggcaaccct gacgcagcaa cgccgcgtga aggatgaagg 60
   ttttcggatt gtaaacttct tttcttaagg acgaaatttg acggtactta aggaataagc 120
   tccggctaac tacgtgcc 138
<210> 206
   <211> 140
   <212> DNA
   <213> Oscillibacter 16S rRNA gene V3 region
<400> 206
   tggggaatat tgggcaatgg gcgcaagcct gacccagcaa cgccgcgtga aggaagaagg 60
   ctttcgggtt gtaaacttct tttaagaggg acgaaggaag tgacggtacc tcttgaataa 120
   gccacggcta actacgtgcc 140
<210> 207
   <211> 137
   <212> DNA
   <213> Clostridiales 16S rRNA gene V3 region
<400> 207
   tgagggatat tggtcaatgg gggaaaccct gaaccagcaa cgccgcgtga gggaagacgg 60
   tcttcggatt gtaaaccttt gtcctctgtg aagataatga cggtagcaga ggaggaagct 120
   ccggctaact acgtgcc 137
<210> 208
   <211> 137
   <212> DNA
   <213> Clostridiales 16S rRNA gene V3 region
<400> 208
   tgggggatat tgcacaatgg gggaaaccct gatgcagcaa cgccgcgtga aggaagaagg 60
   tcttcggatt gtaaactttt gtccttggtg aagataatga cggtagccaa ggaggaagct 120
   ccggctaact acgtgcc 137
<210> 209
   <211> 139
   <212> DNA
   <213> Oscillibacter 16S rRNA gene V3 region
<400> 209
   tggggaatat tgggcaatgg gcgcaagcct gacccagcaa cgccgcgtga aggaagaagg 60
   ctttcgggtt gtaaacttct tttgtcaggg aagagcagaa gacggtacct gacgaataag 120
   ccacggctaa ctacgtgcc 139
<210> 210
   <211> 138
   <212> DNA
   <213> Clostridia 16S rRNA gene V3 region
<400> 210
   tgggggatat tgcacaatgg gggaaaccct gatgcagcga cgccgcgtgt gggaagacgg 60
   tcctctggat tgtaaaccac tgtccccagg gacgaagatg acggtacctg gggaggaagc 120
   tccggctaac tacgtgcc 138
<210> 211
   <211> 138
   <212> DNA
   <213> Ruminococcaceae 16S rRNA gene V3 region
<400> 211
   tggggaatat tgcacaatgg ggggaaccct gatgcagcga tgccgcgtgg aggaagaagg 60
   ttttcggatt gtaaactcct gtcgacagga aagaaaaagg actgtacctg tcaagaaagc 120
   tccggctaac tacgtgcc 138
<210> 212
   <211> 137
   <212> DNA
   <213> Ruminococcus 16S rRNA gene V3 region
<400> 212
   tggggaatat tgcacaatgg ggggaaccct gatgcagcga tgccgcgtgg aggaagaagg 60
   ttttcggatt gtaaactcct gtcttaaagg acgataatga cggtacttta ggaggaagct 120
   ccggctaact acgtgcc 137
<210> 213
   <211> 137
   <212> DNA
   <213> Ruminococcaceae 16S rRNA gene V3 region
<400> 213
   tggggaatat tgcacaatgg aggaaactct gatgcagcga tgccgcgtga gggaagaagg 60
   ttttcggatt gtaaacctct gtcttaaggg acgataatga cggtacctta ggaggaagct 120
   ccggctaact acgtgcc 137
<210> 214
   <211> 137
   <212> DNA
   <213> Ruminococcaceae 16S rRNA gene V3 region
<400> 214
   tgggggatat tgcacaatgg agggaactct gatgcagcga tgccgcgtga gggaagaagg 60
   ttttcggatt gtaaacctct gtggacagag acgataatga cggtatctgt caaggaagcc 120
   acggctaact acgtgcc 137
<210> 215
   <211> 137
   <212> DNA
   <213> Ruminococcaceae 16S rRNA gene V3 region
<400> 215
   tgggggatat tgcacaatgg gggaaaccct gatgcagcga tgccgcgtga gggaagaagg 60
   ttttcggatt gtaaacctct gtggaggggg acgataatga cggtacccct taaggaagcc 120
   acggctaact acgtgcc 137
<210> 216
   <211> 137
   <212> DNA
   <213> Ruminococcaceae 16S rRNA gene V3 region
<400> 216
   tgggggatat tggacaatgg gggaaaccct tatccagcga cgccgcgtga gggaagaagg 60
   ttttcggatt gtaaacctct gtcagcgggg acgataatga cggtacccgc ggaggaagcc 120
   acggctaact acgtgcc 137
<210> 217
   <211> 138
   <212> DNA
   <213> Fastidiosipila 16S rRNA gene V3 region
<400> 217
   tggggaatat tgggcaatgg gcgaaagcct gacccagcga cgccgcgtga aggaagacgg 60
   tcttcggatt gtaaacttta gtactcaggg acgaagaaat gacggtacct gaggttaagc 120
   cacggctaac tacgtgcc 138
<210> 218
   <211> 138
   <212> DNA
   <213> Ruminococcus 16S rRNA gene V3 region
<400> 218
   tgggggatat tgcgcaatgg gggaaaccct gacgcagcaa cgccgcgtga aggaagaagg 60
   ttttcggatt gtaaacttct tttattaagg acgaaagatg acggtactta atgaataagc 120
   tccggctaac tacgtgcc 138
<210> 219
   <211> 139
   <212> DNA
   <213> Oscillibacter 16S rRNA gene V3 region
<400> 219
   tggggaatat tgggcaatgg acgcaagtct gacccagcaa cgccgcgtga aggaagaagg 60
   ctttcgggtt gtaaacttct tttgtcaggg aagagaagaa gacggtacct gacgaacaag 120
   ccacggctaa ctacgtgcc 139
<210> 220
   <211> 139
   <212> DNA
   <213> Oscillibacter 16S rRNA gene V3 region
<400> 220
   tggggaatat tgggcaatgg gcgcaagcct gacccagcaa cgccgcgtga aggaagaagg 60
   ctttcgggtt gtaaacttct tttgacaggg aagagcagaa gacggtacct gtcgaataag 120
   ccacggctaa ctacgtgcc 139
<210> 221
   <211> 138
   <212> DNA
   <213> Ruminococcus 16S rRNA gene V3 region
<400> 221
   tgggggatat tgcgcaatgg gggcaaccct gacgcagcaa cgccgcgtga aggatgaagg 60
   ttttcggatt gtaaacttct tttattaagg acgaattttg acggtactta atgaataagc 120
   tccggctaac tacgtgcc 138
<210> 222
   <211> 139
   <212> DNA
   <213> Oscillibacter 16S rRNA gene V3 region
<400> 222
   tggggaatat tgggcaatgg gcgcaagcct gacccagcaa cgccgcgtga aggaagaagg 60
   ctttcgggtt gtaaacttct tttaagaggg aagagcagaa gacggtacct cttgaataag 120
   ctccggctaa ctacgtgcc 139
<210> 223
   <211> 140
   <212> DNA
   <213> unknown bacterium 16S rRNA gene V3 region
<400> 223
   tggggaatat tgggcaatgg gggaaaccct gacccagcaa cgccgcgtga gggaagaagg 60
   ctttcgggtt gtaaacctct tttaccaggg acgaaggacg tgacggtacc tggagaaaaa 120
   gcaacggcta actacgtgcc 140
<210> 224
   <211> 140
   <212> DNA
   <213> Ruminococcaceae 16S rRNA gene V3 region
<400> 224
   tggggaatat tgggcaatgg gggaaaccct gacccagcaa cgccgcgtga aggaagaagg 60
   ccttcgggtt gtaaacttct tttaccaggg acgaaggacg tgacggtacc tggagaaaaa 120
   gcaacggcta actacgtgcc 140
<210> 225
   <211> 140
   <212> DNA
   <213> Oscillibacter 16S rRNA gene V3 region
<400> 225
   tggggaatat tgggcaatgg gcgaaagcct gacccagcaa cgccgcgtga aggaagaagg 60
   ccttcgggtt gtaaacttct tttaagaggg acgaagaaag tgacggtacc tcttgaataa 120
   gccacggcta actacgtgcc 140
<210> 226
   <211> 140
   <212> DNA
   <213> Fastidiosipila 16S rRNA gene V3 region
<400> 226
   tggggaatat tgggcaatgg gcgcaagcct gacccagcaa cgccgcgtga aggaagaagg 60
   ctttcgggtt gtaaacttct tttgagaggg acgaaacaaa tgacggtacc tcttgaataa 120
   gccacggcta actacgtgcc 140
<210> 227
   <211> 140
   <212> DNA
   <213> Oscillibacter 16S rRNA gene V3 region
<400> 227
   tggggaatat tgggcaatgg gcggaagcct gacccagcaa cgccgcgtga aggaagaagg 60
   ctttcgggtt gtaaacttct tttcttgggg acgaagaaag tgacggtacc caaggaataa 120
   gccacggcta actacgtgcc 140
<210> 228
   <211> 140
   <212> DNA
   <213> Oscillibacter 16S rRNA gene V3 region
<400> 228
   tggggaatat tgggcaatgg gcgcaagcct gacccagcaa cgccgcgtga aggaagaagg 60
   ctttcgggtt gtaaacttct tttgaggggg acgaaggatg tgacggtacc ccttgaataa 120
   gccacggcta actacgtgcc 140
<210> 229
   <211> 140
   <212> DNA
   <213> Oscillibacter 16S rRNA gene V3 region
<400> 229
   tggggaatat tgggcaatgg gcgcaagcct gacccagcaa cgccgcgtga aggaagaagg 60
   ctttcgggtt gtaaacttct tttcttgggg acgaagaaag tgacggtacc cgaggaataa 120
   gccacggcta actacgtgcc 140
<210> 230
   <211> 137
   <212> DNA
   <213> Marvinbryantia 16S rRNA gene V3 region
<400> 230
   tggggaatat tgcacaatgg gggaaaccct gatgcagcga cgccgcgtgg gtgaagaagt 60
   atttcggtat gtaaagccct atcagcaggg aagatcatga cggtacctga ctaagaagcc 120
   ccggctaact acgtgcc 137
<210> 231
   <211> 140
   <212> DNA
   <213> Oscillibacter 16S rRNA gene V3 region
<400> 231
   tggggaatat tgggcaatgg gcgcaagcct gacccagcaa cgccgcgtga aggaagaagg 60
   ctttcgggtt gtaaacttct tttctgaggg acgaagcaag tgacggtacc ttaggaataa 120
   gccacggcta actacgtgcc 140
<210> 232
   <211> 139
   <212> DNA
   <213> Ruminococcaceae 16S rRNA gene V3 region
<400> 232
   tggggaatat tgggcaatgg gcgcaagcct gacccagcaa cgccgcgtga aggaagaagg 60
   ctttcgggtt gtaaacttct tttgacaggg aagaggagaa gacggtacct gtcgaataag 120
   ctccggctaa ctacgtgcc 139
<210> 233
   <211> 139
   <212> DNA
   <213> Ruminococcaceae 16S rRNA gene V3 region
<400> 233
   tggggaatat tgggcaatgg gcgcaagcct gacccagcaa cgccgcgtga aggaagaagg 60
   ccctcgggtt gtaaacttct tttatcaggg acgaagaagt gacggtacct gatgaataag 120
   ccacggctaa ctacgtgcc 139
<210> 234
   <211> 138
   <212> DNA
   <213> Clostridiales 16S rRNA gene V3 region
<400> 234
   tggggaatat tgcgcaatgg gggaaaccct gacgcagcaa cgccgcgtga ttgaagaagg 60
   ccttcgggtt gtaaagatct ttaatcgggg acgaattttg acggtacccg aagaataagc 120
   tccggctaac tacgtgcc 138
<210> 235
   <211> 138
   <212> DNA
   <213> Clostridiales 16S rRNA gene V3 region
<400> 235
   tggggaatat tgcgcaatgg gggaaaccct gacgcagcaa cgccgcgtga ttgaagaagg 60
   ccctcgggtt gtaaagatct ttaatcgggg acgaagaatg acggtacccg aagaataagc 120
   tccggctaac tacgtgcc 138
<210> 236
   <211> 138
   <212> DNA
   <213> Clostridiales 16S rRNA gene V3 region
<400> 236
   tggggaatat tgcgcaatgg gggaaaccct gacgcagcaa cgccgcgtga gtgaagaagg 60
   ccttcgggtt gtaaagctct ttaatcaggg acgaagaacg acggtacctg aagaataagc 120
   tccggctaac tacgtgcc 138
<210> 237
   <211> 140
   <212> DNA
   <213> Firmicutes 16S rRNA gene V3 region
<400> 237
   tggggaatat tgggcaatgg gcgaaagcct gacccagcaa cgccgcgtga aggaagaagg      60
   tcttcggatt gtaaacttct tttatcaggg acgaaggaag tgacggtacc tgatgaataa     120
   gccacggcta actacgtgcc                                                 140
<210> 238
   <211> 137
   <212> DNA
   <213> unknown bacterium 16S rRNA gene V3 region
<400> 238
   tcgggaatat tgcgcaatgg aggcaactct gacgcagtga cgccgcgtat aggaagaagg      60
   ttttcggatt gtaaactatt gtccacaggg aagaaaagga ctgtacctgt gaagaaagct     120
   ccggctaact acgtgcc                                                    137
<210> 239
   <211> 138
   <212> DNA
   <213> Firmicutes 16S rRNA gene V3 region
<400> 239
   tcgggaatat tgcgcaatgg aggaaactct gacgcagtga cgccgcgtat aggaagaagg      60
   ttttcggatt gtaaactatt gtccacaggg aagataaaag actgtacctg tgaagaaagc     120
   tccggctaac tacgtgcc                                                   138
<210> 240
   <211> 138
   <212> DNA
   <213> unknown bacterium 16S rRNA gene V3 region
<400> 240
   tcgggaatat tgcgcaatgg aggaaactct gacgcagtga cgccgcgtat aggaagaagg      60
   ttttcggatt gtaaactatt gtcgataggg aagaaaaaag actgtaccta tcaagaaagc     120
   tccggctaac tacgtgcc                                                   138
<210> 241
   <211> 138
   <212> DNA
   <213> Clostridia 16S rRNA gene V3 region
<400> 241
   tcgggaatat tgcgcaatgg agggaactct gacgcagtga cgccgcgtat aggaagaagg 60
   ttttcggatt gtaaactatt ttagtcaggg aagaaagcag acggtacctg aagaataagc 120
   tccggctaac tacgtgcc 138
<210> 242
   <211> 138
   <212> DNA
   <213> unknown bacterium 16S rRNA gene V3 region
<400> 242
   tcgggaatat tgcacaatgg aggaaactct gatgcagtga cgccgcgtgc aggaagaagg 60
   ttttcggatt gtaaactgct ttagacaggg aagaaaaaag acagtacctg tagaataagc 120
   tccggctaac tacgtgcc 138
<210> 243
   <211> 138
   <212> DNA
   <213> unknown bacterium 16S rRNA gene V3 region
<400> 243
   tcgggaatat tgcgcaatgg aggaaactct gacgcagtga cgccgcgtat aggaagaagt 60
   ttttcggaat gtaaactatt gtcgttaggg aagagaaagg acagtaccta aggaggaagc 120
   tccggctaac tacgtgcc 138
<210> 244
   <211> 138
   <212> DNA
   <213> Clostridia 16S rRNA gene V3 region
<400> 244
   tcgggaatat tgcgcaatgg agggaactct gacgcagtga cgccgcgtat aggaagaagg 60
   ttttcggatt gtaaactatt gtcgttaggg aagaaaaaag acagtaccta aggaggaagc 120
   cccggctaac tatgtgcc 138
<210> 245
   <211> 138
   <212> DNA
   <213> unknown bacterium 16S rRNA gene V3 region
<400> 245
   tcgggaatat tgcgcaatgg aggaaactct gacgcagtga cgccgcgtat aggaagaagg 60
   tcttcggatt gtaaactatt gtcgttaggg aagagaaagg acagtaccta aggaggaagc 120
   tccggctaac tacgtgcc 138
<210> 246
   <211> 140
   <212> DNA
   <213> unknown bacterium 16S rRNA gene V3 region
<400> 246
   tcgggaatat tgcgcaatgg aggaaactct gacgcagtga cgccgcgtgc aggaagaagg 60
   ttttcggatt gtaaactgct ttagacaggg aagaaacaaa tgacagtacc tgtagaataa 120
   gctccggcta actacgtgcc 140
<210> 247
   <211> 137
   <212> DNA
   <213> unknown bacterium 16S rRNA gene V3 region
<400> 247
   tcgggaatat tgcgcaatgg aggaaactct gacgcagtga cgccgcgtat aggaagaagg 60
   ttttcggatt gtaaactatt gtcgtgaggg aagaaattga cagtacctca ggaggaagct 120
   ccggctaact atgtgcc 137
<210> 248
   <211> 138
   <212> DNA
   <213> unknown bacterium 16S rRNA gene V3 region
<400> 248
   tcgggaatat tgcgcaatgg aggaaactct gacgcagtga cgccgcgtat aggaagaagg 60
   ttttcggatt gtaaactatt gtcgttaggg aagagaaagg acagtaccta aggaggaagc 120
   tccggctaac tacgtgcc 138
<210> 249
   <211> 140
   <212> DNA
   <213> unknown bacterium 16S rRNA gene V3 region
<400> 249
   tcgggaatat tgcgcaatgg aggaaactct gacgcagtga cgccgcgtgc aggaagaagg 60
   ttttcggatt gtaaactgct ttagacaggg aagaaagaaa tgacggtacc tgtagaataa 120
   gctccggcta actacgtgcc 140
<210> 250
   <211> 138
   <212> DNA
   <213> unknown bacterium 16S rRNA gene V3 region
<400> 250
   tcgggaatat tgcgcaatgg aggaaactct gacgcagtga cgccgcgtat aggaagaagg 60
   ttttcggatt gtaaactatt gtcgttaggg aagataaaag actgtaccta aggaggaagc 120
   cccggctaac tatgtgcc 138
<210> 251
   <211> 138
   <212> DNA
   <213> unknown bacterium 16S rRNA gene V3 region
<400> 251
   tcgggaatat tgcgcaatgg aggaaactct gacgcagtga cgccgcgtat aggaagaagt 60
   ttttcggaat gtaaactatt gtcattaggg aagagaaagg acggtaccta aggaggaagc 120
   cccggctaac tatgtgcc 138
<210> 252
   <211> 138
   <212> DNA
   <213> unknown bacterium 16S rRNA gene V3 region
<400> 252
   tcgggaatat tgcgcaatgg aggaaactct gacgcagtga cgccgcgtat aggaagaagg 60
   ttttcggatt gtaaactatt gtcgttaggg aagaaaaaag acagtaccta aggaggaagc 120
   cccggctaac tatgtgcc 138
<210> 253
   <211> 139
   <212> DNA
   <213> unknown bacterium 16S rRNA gene V3 region
<400> 253
   ttgggaatat tggacaatgg aggaaactct gatccagtga cgccgcgtga aggaagaagg 60
   tcttcggatt gtaaacttat tttgtcaggg aagaataaat gactgtacct gaagaaaaag 120
   caccggctaa ctacgtgcc 139
<210> 254
   <211> 140
   <212> DNA
   <213> Clostridiales 16S rRNA gene V3 region
<400> 254
   tggggaatat tgcgcaatgg gggaaaccct gacgcagcaa cgccgcgtgc aggaagaagg 60
   tcttcggatt gtaaactgtt gtcgcagggg aagaagacag tgacggtacc ctgtgagaaa 120
   gtcacggcta actacgtgcc 140
<210> 255
   <211> 139
   <212> DNA
   <213> unknown bacterium 16S rRNA gene V3 region
<400> 255
   tggggaatat tgggcaatgg gcgaaagcct gacccagcaa cgccgcgtgg aggaagaagg 60
   ttttcggatc gtaaactcct gtcctaagag acgaggaaga gacggtatct taggaggaag 120
   ccccggctaa ctacgtgcc 139
<210> 256
   <211> 140
   <212> DNA
   <213> unknown bacterium 16S rRNA gene V3 region
<400> 256
   tggggaatat tgggcaatgg gcgaaagcct gacccagcaa cgccgcgtga gggaagaagg 60
   ttttcggatt gtaaacctct gtcctaagtg acgaaggaag tgacggtagc ttaggaggaa 120
   gccccggcta actacgtgcc 140
<210> 257
   <211> 140
   <212> DNA
   <213> unknown bacterium 16S rRNA gene V3 region
<400> 257
   tggggaatat tgggcaatgg gcgaaagcct tacccagcaa cgccgcgtga gggaagaagg 60
   ttttcggatt gtaaacctct gtcctggggg acgaaggaag tgacggtacc ccgggaggaa 120
   gccccggcta actacgtgcc 140
<210> 258
   <211> 137
   <212> DNA
   <213> Clostridiales 16S rRNA gene V3 region
<400> 258
   tggggaatat tgggcaatgg gggaaaccct gacccagcaa cgccgcgtga aggaagaagg 60
   ttttcggatc gtaaacttct atccttggtg aaaatgatga tggtagccaa gaaggaagcc 120
   ccggctaact acgtgcc 137
<210> 259
   <211> 140
   <212> DNA
   <213> unknown bacterium 16S rRNA gene V3 region
<400> 259
   tggggaatat tgggcaatgg gcgaaagcct gacccagcaa cgccgcgtga gggaagaagg 60
   gtttcggctc gtaaacctct gtcctatggg acgaaggaag tgacggtacc ataggaggaa 120
   gccccggcta actacgtgcc 140
<210> 260
   <211> 137
   <212> DNA
   <213> Clostridiales 16S rRNA gene V3 region
<400> 260
   tggggaatat tgcacaatgg gcgaaagcct gatgcagcaa cgccgcgtga gcgaagaagg 60
   tcttcggatc gtaaagctct gtccttgggg aagataatga cggtacccaa ggaggaagcc 120
   ccggctaact acgtgcc 137
<210> 261
   <211> 140
   <212> DNA
   <213> unknown bacterium 16S rRNA gene V3 region
<400> 261
   tggggaatat tgggcaatgg aggcaactct gacccagcaa cgccgcgtga atgaagaagg 60
   tcctaggatt gtaaagttct tttatgatag acgaataaaa tgacggtata tcatgaataa 120
   gccacggcta actacgtgcc 140
<210> 262
   <211> 140
   <212> DNA
   <213> unknown bacterium 16S rRNA gene V3 region
<400> 262
   tggggaatat tgggcaatgg aggcaactct gacccagcaa cgccgcgtga atgaagaagg 60
   ccttcgggtt gtaaagttct ttaatggggg acgaagaaag tgacggtacc ccaagaataa 120
   gccacggcta actacgtgcc 140
<210> 263
   <211> 137
   <212> DNA
   <213> Lachnospiraceae 16S rRNA gene V3 region
<400> 263
   tgggggatat tgcacaatgg gggaaaccct gatgcagcga cgccgcgtga gtgaagaagt 60
   atttcggtat gtaaagctct atcagcaggg aagaaaatga cggtacctga ctaagaagcc 120
   ccggctaact acgtgcc 137
<210> 264
   <211> 163
   <212> DNA
   <213> Firmicutes 16S rRNA gene V3 region
<400> 264
   ttaggaatat tcgtcaatgg gggaaaccct gaacgagcaa tgccgcgtga gtgatgacgg 60
   tctttatgat tgtaaaactc tgttgtaagg aaagaaccct tatcatagga aatgatgata 120
   agttgacggt accttaccag aaagccccgg ctaactacgt gcc 163
<210> 265
   <211> 146
   <212> DNA
   <213> Anaeroplasma 16S rRNA gene V3 region
<400> 265
   tagggaattt tcggcaatgg ggggaaccct gaccgagcaa cgccgcgtga acgaagaagt 60
   tattcgtaat gtaaagttct tttatcaggg aagaaaagaa gggaattgac ggtacctgat 120
   gaataagctc cggctaacta cgtgcc 146
<210> 266
   <211> 162
   <212> DNA
   <213> Clostridiales 16S rRNA gene V3 region
<400> 266
   tggggaatct tccgcaatgg gcgaaagcct gacggagcga cgccgcgtga gtgaagaagg 60
   tcttcggacc gtaaagctct tttgttgcag gcgaaaggac ttaagaggaa atgcttaagt 120
   taagacggta tggaacgaat aagccacggc taactacgtg cc 162
<210> 267
   <211> 137
   <212> DNA
   <213> TM7_genera_incertae_sedis 16S rRNA gene V3 region
<400> 267
   tagggaatct ttcacaatgg gcgaaagcct gatggagcaa cgccgcgtgc aggatgaagg 60
   ccttcgggtt gtaaactgct tttataagcg agaaatatga tggtaactta tgaataagga 120
   tcggctaact acgtgcc 137
<210> 268
   <211> 140
   <212> DNA
   <213> unknown bacterium 16S rRNA gene V3 region
<400> 268
   tggggaattt tggacaatgg acggaagtct gatccagcaa cgcagcgtga aggacgaagg       60
   ttctcggatt gtaaacttct tttgcagggg aagaaaaaaa tgacggtacc ctgtgaataa      120
   gccacggcta actacgtgcc                                                  140

## Description

### TECHNICAL FIELD

The present application relates to compositions and methods for improving gut microbiota populations and related application in drugs, nutritional supplements, health care products, food and beverage.

### BACKGROUND

Unless otherwise indicated herein, the materials described in this section are not prior art to the claims in this application and are not admitted to be prior art by inclusion in this section.

Inside the human body lives a large number of symbiotic microbes, among which the gut microbiota acts as an important environmental factor to host health. There are more than 1000 species of bacteria, whose number exceeds 10 folds of the number of human cells, and whose gene number is about 150 folds of that in human cells. In this context, the human body is considered as a "superorganism" made of host cells and symbiotic microbes including gut microbiota, and the genome encoding a consortium of gut microbes (microbiome) as the second human genome, also known as "human metagenome". When human health status changes, the composition of symbiotic microbes changes accordingly. Conversely, changes in the composition of symbiotic microbes lead to the change in the human health status. Together, the diversity in human genome and the genome of gut microbiome affects immunity, nutrition, metabolism, and the health and disease status of the human host. However, up to now, it is not clear by what mechanisms the gut microbiota contribute to disease etiology and pathology, which type of bacteria is positively correlated with the health status of the host, and which type of bacteria is negatively correlated with the health status of the host.

US 2011/0281852 A1 describes treatment and prevention of weight gain and obesity with berberine and/or berberine analogs and a pharmacologic agent. US 6,551,628 B1 describes a herbal formulation comprising a solid component such as berberine sulphate as an intestinal tract cleanser. CN 102327309 A discloses a berberine extract for preparing PPAR and LXR alpha agonists utilized for treating diabetes, diabetic hyperlipidemia, hypercholesterolemia, diabetes diabetic retinopathy and obesity. CN 101524404 A discloses intestinal sustained-released and controlled-release capsules, wherein curative medicines in medicinal capsules are Berberis prunosa and Radix Scutellariae extracts in combination. Med Sci Monit, 2011; 17(7); RA164-167 (Junling Han et al) is a review article titled Modulating gut microbiota as an anti-diabetic mechanism of berberine. Journal of Pharmacy and Pharmacology, 2010, 62:440-447 (Shan-Shan Xie et al) describes the effect of HLJDD (Huang-Lian-Jie-Du-Decoction consisting of Rhizoma Coptidis, Radix Scutellariae, Cortex Phellodendri and Fructus Gardeniae) on the pharmacokinetic behaviour of verapamil in rats. British Journal of Nutrition, 2012, S1-S13 (Thomas et al.) provides a review of the impact of the intestinal microbiota.

### SUMMARY

The present invention is defined in and by the appended claims.

The following summary is illustrative only and is not intended to be in any way limiting. In addition to the illustrative aspects, embodiments, and features described above, further aspects, embodiments, and features will become apparent by reference to the drawings and the following detailed description.

The application provides methods for improving gut microbiota population. The method includes administering to a subject a composition to increase a first gut microbiota population while simultaneously decrease a second gut microbiota population in the subject. The first gut microbiota population may include a short-chain fatty acid (SCFA)-producing bacterium. The second gut microbiota population may include an endotoxin-producing bacterium.

The application also provides methods for screening a test compound that may be active in improving gut microbiota population. The method includes administering to a control subject an effective amount of a control composition to increase a first gut microbiota population while simultaneously decrease a second gut microbiota population in the control subject, administering to a test subject an amount of a test compound, and comparing the gut microbiota population of the controlled subject with the gut microbiota population of the test subject. A similarity of at least about 80% is indicative that the test compound is active in improving gut microbiota population. The first gut microbiota population may include a short-chain fatty acid (SCFA)-producing bacteria. The second gut microbiota population may include an endotoxin-producing bacterium.

The application also provides compositions for improving gut microbiota population. The composition is capable of selectively increasing a first gut microbiota population while simultaneously decreasing a second gut microbiota population in a subject. The first gut microbiota population may include a short-chain fatty acid (SCFA)-producing bacteria. The second gut microbiota population may include an endotoxin-producing bacterium. The composition is administered to the subject at a dosage of from about 50 mg/kg body weight to about 400 mg/body weight and with once a day administration for at least two weeks.

### BRIEF DESCRIPTION OF THE DRAWINGS

The foregoing and other features of this disclosure will become more fully apparent from the following description and appended claims, taken in conjunction with the accompanying drawings. Understanding that these drawings depict only several embodiments arranged in accordance with the disclosure and are, therefore, not to be considered limiting of its scope, the disclosure will be described with additional specificity and detail through use of the accompanying drawings, in which:

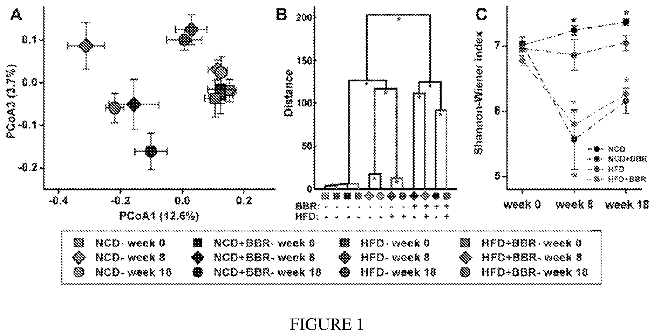

FIGURE 1 illustrates the effect of berberine on the rat gut microbiota structure; FIGURE 1A is the PCoA score plot of changes in the rat gut microbiota structure in response to HFD feeding and berberine administration; FIGURE 1B shows the clustering of the gut microbiota based on mahalanobis distances calculated using MANOVA; and FIGURE 1C shows the Shannon-Wiener index, calculated after rarefying to an equal number of sequence reads for all samples;

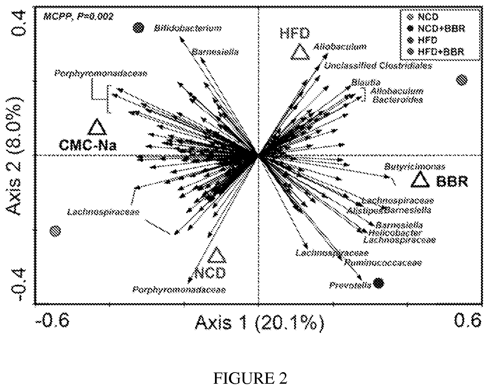

FIGURE 2 illustrates some representative differences in the gut microbiota structure caused by berberine treatment using distance triplot of redundancy analysis (RDA) of gut microbiota;

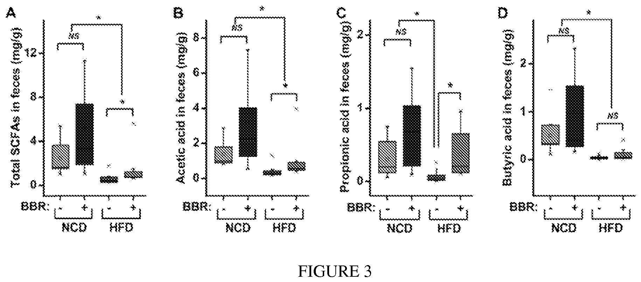

FIGURE 3 illustrates the effect on the short-chain fatty acid contents in rat feces when the rats were fed with high fat diet or normal chow diet; FIGURE 3A shows the levels of total short-chain fatty acids (SCFAs); FIGURE 3B shows the level of acetic acid; FIGURE 3C shows the level of propionic acid; and FIGURE 3D shows the level of butyric acid (D);

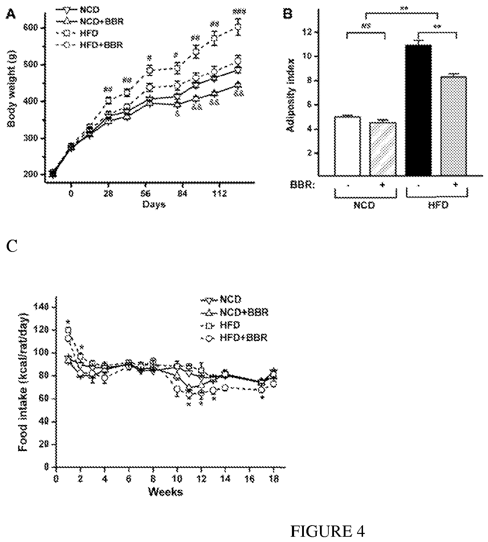

FIGURE 4 illustrates the effect of berberine on obesity phenotypes and food intake in rats; FIGURE 4A shows the effect of berberine on the body weight gain; FIGURE 4B shows the effect of berberine on the adiposity index, calculated as the fat pad weight (sum of epididymal and perirenal fat pads) per 100 g of total body weight; and FIGURE 4C shows the food intake of rats during the entire trial;

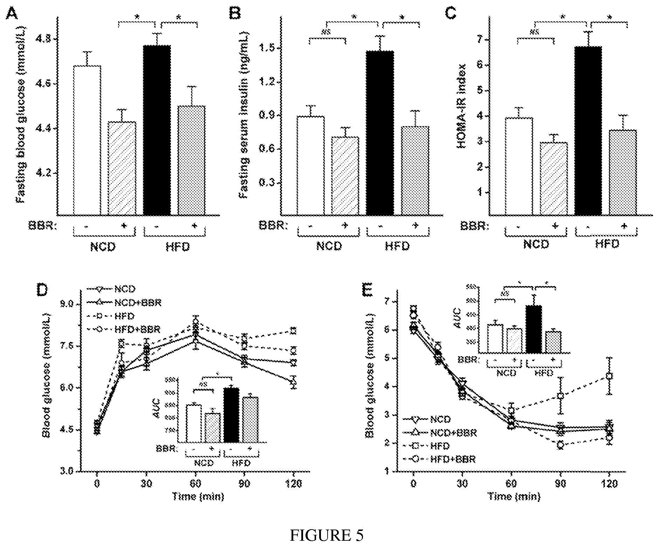

FIGURE 5 illustrates the effect of berberine on the insulin sensitivity in rats; FIGURE 5A shows the effect of berberine on fasting blood glucose (FBG); FIGURE 5B shows the effect of berberine on fasting serum insulin (FINS); FIGURE 5C shows the effect of berberine on homeostasis assessment of insulin resistance (HOMA-IR) value, calculated according to the formula fasting insulin (µU/mL) × fasting glucose (mmol/L)/22.5; FIGURE 5D shows the effect of berberine on oral glucose tolerance test (OGTT); and FIGURE 5E shows the effect of berberine on intra-peritoneal insulin tolerance test (ITT); and

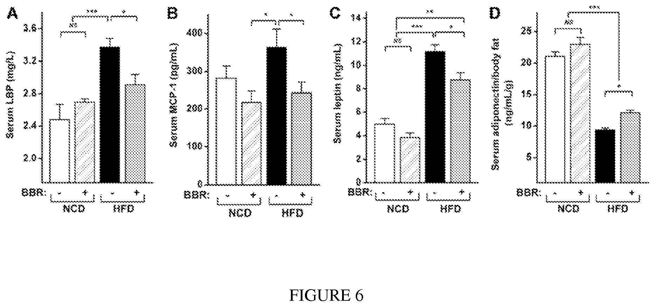

FIGURE 6 illustrates the effect of berberine on the level of inflammation factors in rats; FIGURE 6A shows the effect of berberine on serum lipopolysaccharide (LPS)-binding protein (LBP); FIGURE 6B shows the effect of berberine on serum leptin; FIGURE 6C shows the effect of berberine on serum MCP-1; and FIGURE 6D shows the effect of berberine on serum adiponectin corrected for body fat.

### DETAILED DESCRIPTION

In the following detailed description, reference is made to the accompanying drawings, which form a part hereof. In the drawings, similar symbols typically identify similar components, unless context dictates otherwise. The illustrative examples described in the detailed description, and drawings are not meant to be limiting. Other embodiments may be utilized, and other changes may be made, without departing from the scope of the claims. It will be readily understood that the aspects of the present disclosure, as generally described herein, and illustrated in the Figures, can be arranged, substituted, combined, separated, and designed in a wide variety of different configurations, all of which are explicitly contemplated herein.

This application is generally drawn, *inter alia,* to compositions, methods, processes, apparatus, systems, devices, and/or products related to improving gut microbiota population.

The application provides novel compositions and methods for improving gut microbiota population. The application identifies bacteria families that are closely related to host metabolism utilizing, for example, high throughput sequencing and multivariate statistical methods.

The application provides methods for improving gut microbiota population. The method may include selectively enriching a first gut microbiota population. The first gut microbiota population may include, for example, an short-chain fatty acid (SCFA)-producing bacterium. The method may include suppressing a second gut microbiota population. The second gut microbiota population may include, for example, an endotoxin-producing bacterium.

The method may include the step of administering to a subject a composition. The composition may be an oral or parenteral formulation. The composition may selectively increase the first gut microbiota population while simultaneously decrease the second gut microbiota population.

The enrichment of the first gut microbiota population and suppression of the second microbiota population may be carried out simultaneously. The method may result in the prevention or treatment of metabolic syndrome including, without limitation, obesity, diabetes mellitus, insulin resistance, hyperlipoproteinemia, hyperuricemia, hepatic steatosis, hypercholesterolemia, hypertriglyceridemia, inflammation, and other disorders.

The short-chain fatty acid (SCFA)-producing bacteria inside gut are mostly beneficial bacteria. These bacteria either directly or, by increasing short-chain fatty acid inside gut, thus indirectly, perform functions including without limitation anti-inflammation, protecting intestinal barrier function, and regulating metabolism and immune system. These functions lead to the prevention or treatment of obesity, insulin resistance, diabetes, and other metabolic diseases.

Using the disclosed methods for improving gut microbiota population, the increased gut microbiota population may include *Alistipes, Allobaculum, Bacteroides, Barnesiella, Blautia, Butyricicoccus, Butyricimonas, Dorea, Helicobacter, Hespellia, Holdemania, Lawsonia, Oscillibacter, Parabacteroides, Phascolarctobacterium, Prevotella,* or *Sedimentibacter.* In one embodiment, the increased gut microbiota population may include *Bacteroidaceae, Coriobacteriaceae, Desulfovibrionaceae, Erysipelotrichaceae, Flavobacteriaceae, Helicobacteracea, Incertae Sedis XI, Incertae Sedis XIV, Lachnospiraceae, Porphyromonadaceae, Prevotellaceae, Rikenellaceae, Ruminococcaceae,* or *Veillonellaceae;* alternatively, the increased gut bacteria comprise *Campylobacterales, Desulfovibrionales, Bacteroidales, Coriobacteriales, Flavobacteriales, Clostridiales,* or *Erysipelotrichales;* alternatively, the increased gut bacteria comprise *Epsilonproteobacteria, Deltaproteobacteria, Bacteroidia, Coriobacteridae, Flavobacteria, Clostridia, or Erysipelotrichi;* alternatively, the increased gut bacteria comprise *Proteobacteria, Bacteroidetes, Actinobacteria,* or *Firmicutes.*

For example, the increased gut microbiota population may include a bacterium whose V3 region of 16S rRNA gene sequence has at least about 70%, at least about 75%, at least about 80%, at least about 85%, at least about 90%, at least about 95%, at least about 98%, or at least about 99% similarity with a nucleic acid sequence selected from a group consisting of SEQ ID NO: 1-93.

The endotoxin-producing bacteria inside gut are mostly harmful bacteria, which, either directly or indirectly by increasing endotoxin, promote inflammation, damage intestinal barrier function, and increase disorder in metabolism and immune system, as a result inducing obesity, insulin resistance, diabetes and other metabolic diseases.

According to the methods for improving gut microbiota population disclosed herein, the decreased gut bacteria may include *Alistipes, Anaeroplasma, Barnesiella, Bifidobacterium, Butyricimonas, Butyrivibrio, Coprococcus, Fastidiosipila, Helicobacter, Hespellia, Marvinbryantia, Oribacterium, Oscillibacter, Prevotella, Roseburia, Ruminococcus,* or *TM7 genera incertae sedis;* alternatively, the decreased gut bacteria comprise *Helicobacteraceae, Lachnospiraceae, Porphyromonadaceae, Prevotellaceae, Rikenellaceae, Ruminococcaceae, Anaeroplasmataceae,* or *Bifidobacteriaceae.* In one embodiment, the decreased gut microbiota population may include *Campylobacterales, Bacteroidales, Clostridiales, Anaeroplasmatales,* or *Bifidobacteriales;* alternatively, the decreased gut bacteria may include *Epsilonproteobacteria, Alphaaproteobacteria, Bacteroidia, Clostridia, Actinobacteridae,* or *Mollicutes.* In one embodiment, the decreased gut microbiota population may include *Proteobacteria, Bacteroidetes, Actinobacteria, Firmicutes,* or *Tenericutes.*

For example, the decreased gut bacteria may include a bacteria whose V3 region of 16S rRNA gene sequence has at least about 70%, at least about 75%, at least about 80%, at least about 85%, at least about 90%, at least about 95%, at least about 98%, or at least about 99% similarity with a nucleic acid sequence selected from a group consisting of SEQ ID NO: 94-268.

The methods may cause decrease of the serum level of pro-inflammatory factors. Example pro-inflammatory factors include without limitation cytokines such as lipopolysaccharide-binding protein, monocyte chemoattractant protein-1, or leptin. Additionally and optionally, the methods may increase the serum level of cytokine such as adiponectin.

The level of gut short-chain fatty acids may be increased using the disclosed methods. The short-chain fatty acids may include without limitation acetic acid, propionic acid, butyric acid, valeric acid, isobutyric acid, and isovaleric acid.

To improve the gut microbiota population, the compositions may be administered orally or parentally. In one embodiment, the composition may be a food, a drink, a supplement, or a pharmaceutical formulation. In one embodiment, the composition may be in the form of suppository, tablet, pill, granule, powder, film, microcapsule, aerosol, spirit, tincture, tonic, liquid suspension, or syrup. The composition may be administered at a dosage of from about 50 mg/kg body weight to about 400 mg/body weight.

In one example, the composition including berberine was administered to a subject. The gut microbiota population of the subject was then analyzed using 454 pyrosequencing techniques and the level of short-chain fatty acid was assayed by gas chromatography. The results demonstrated that berberine is capable of altering gut microbiota population: enriching certain bacteria including those that produce short-chain fatty acid while simultaneously suppressing or eliminating certain bacteria including those that produce endotoxin. It is further demonstrated that berberine administered orally is capable of increasing the level of shot-chain fatty acid inside the gut, and the increase is more pronounced in individuals who have metabolic syndrome. Additional experimentation and observation further demonstrated that above described effects by berberine on the gut microbiota population have beneficial results including without limitation improving insulin sensitivity, reducing inflammation, controlling weight gain, and preventing obesity induced by over-eating, chronic inflammation and insulin resistance.

The application also provides methods for screening drugs, compounds, compositions, extracts, or formulations capable of improving gut microbiota population. The methods may be used in the development of drugs, nutritional supplements, health care products, food, and beverages that improve health and/or prevent obesity or other related metabolic syndromes by targeting gut microbiota population in a subject.

The screening method may include administering to a control subject an effective amount of a control composition to increase the first gut microbiota population while simultaneously decrease the second gut microbiota population in the control subject, administering to a test subject an amount of a test composition, and comparing the gut microbiota population of the controlled subject and the gut microbiota population of the test subject.

As used herein, the term "subject" refers to an animal, such as a mammal, for example a human. In some embodiments, the subject may be a rat. In some embodiments, the subject may be a mouse, In some embodiment, the subject may be a human.

If the test compound demonstrates similar effect on gut microbiota as the control, the test compound is active in improving gut microbiota population. As used therein, "similar" refers to a similarity of at least about 75%, at least about 80%, at least about 90%, at least about 95%, at least about 98%, or at least about 99%.

The test composition may be a single compound, a mixture, a food, a food additive, a nutritional supplement, health care product, a pharmaceutical formulation, or a beverage.

The application provides compositions for improving gut microbiota population. The composition may be capable of selectively increasing the first gut microbiota population while simultaneously decreasing the second gut microbiota population in a subject.

The composition may include a chemical compound, a natural medicine, a natural product, or an herbal extract. In one embodiment, the chemical compound may include, without limitation, berberine, berberine derivatives, isoquinoline alkaloids, or any combinations thereof.

The composition may include natural medicine, whole, fragmented or powdered herb, or herbal extract derived from plants of *Berberis, Coptis, Scutellaria, Phellodendron, Momordica, Ilex, Sophora, Gentiana, Anemarrhena, Gardenia* , *Rheum,* or *Taraxacum.* In one embodiment, , the composition may include natural medicine, whole, fragmented or powdered herb, or herbal extract derived from plants of *Berberidaceae, Ranunculaceae, Lamiaceae, Rutaceae, Cucurbitacea, Aquifoliaceae, Leguminosae, Gentianaceae, Agavaceae, Rubiaceae, Polygonaceae, Asteraceae, Menispermaceae,* or *Cucurbitaceae.* In one embodiment, the composition may include natural medicine, whole, fragmented or powdered herb, or herbal extract derived from *Berberis vulgaris, Coptis chinensis, Scutellaria baicalensis, Phellodendri Chinensis, Momordica charantia, Ilex kudingcha, Sophora flavescens, Gentiana scabra, Anemarrhena asphodeloides, Gardenia jasminoides, Rheum palmatum,* or *Herba Taraxaci.*

The following examples are for illustration only and are not intended to limit the scope of the claims.

### EXAMPLES

These examples use Wistar rats (8 weeks old, male) as testing subjects and berberine as a representative compound. 40 Wistar rats were acclimatized for two weeks and were subsequently randomly divided into four groups: normal diet group (NCD), normal diet plus berberine treatment (NCD+BBR), high fat diet (HFD), and high fat diet plus berberine treatment (HFD+BBR). Each group continued for another 18 weeks. During these 18 weeks, the NCD+BBR group received intragastric administration of berberine at stated levels. Feces samples from each animal were collected at various time points. 454 pyrosequencing was performed to analyze gut microbiota structure. Gas chromatography was used to assay the short-chain fatty acid level of the feces. In addition, weight, food intake, insulin sensitivity, systemic inflammation levels were monitored and measured during the 18-week period.

### Berberine Altered the Gut Microbiota Population Under Both Normal Diet and High Fat Diet Conditions

The gut microbiota populations in rats from all four experimental groups were analyzed by 454 pyrosequencing and multivariate statistical analyses. The results, PCoA analyses based on unweighted Unifrac distance, demonstrated that berberine altered gut microbiota structure in both the normal diet group and high fat diet group at a statistically significant level; and berberine accounts for 12.6% of overall changes in the gut microbiota population (FIGURE 1A). Diet also exerted statistically significant influence on gut microbiota population. FIGURE 1A reveals a statistically significant difference (3.7%) in gut microbiota population between two different diets (NCD versus HFD) along the vertical axis. The multivariate variance analysis (MANOVA) on the four groups demonstrated that berberine or diet has a statistically significant influence on the gut microbiota (P<0.01), but the most pronounced difference derives from the presence of berberine (FIGURE 1B). Shannon-Wiener parameter indicates that berberine reduces diversity of gut microbiota population at a statistically significant level (P<0.05).

### Berberine Enriches the Short-Chain Fatty Acid-Producing Bacteria and Reduces Endotoxin-Producing Bacteria in Gut Microbiota Population

268 berberine-related OTU were identified by redundancy analysis (RDA) and the detailed results are shown in FIGURE 2, TABLE 1, and TABLE 2. 93 OTU (SEQ_ID_NO 1-93) (TABLE 1) are enriched by berberine, while 175 OTU (SEQ_ID_NO 94-268) (TABLE 2) were suppressed or eliminated.

The taxonomy of these 268 OTU was analyzed using RDP classifier with representative sequence of the OTU. It was revealed that the berberine-suppressed bacteria included those of *Alistipes, Anaeroplasma, Barnesiella, Bifidobacterium, Butyricimonas, Butyrivibrio, Coprococcus, Fastidiosipila, Helicobacter, Hespellia, Marvinbryantia, Oribacterium, Oscillibacter, Prevotella, Roseburia, Ruminococcus, TM7_genera_incertae_sedis,* and others. Among them, the *Helicobacter,* belonging to Proteobacteria phylum, is capable of producing highly active endotoxin. In addition, it was revealed that the berberine-increased bacteria included those of *Alistipes, Allobaculum, Bacteroides, Barnesiella, Blautia, Butyricicoccus, Butyricimonas, Dorea, Helicobacter, Hespellia, Holdemania, Lawsonia, Oscillibacter, Parabacteroides, Phascolarctobacterium, Prevotella,* and *Sedimentibacter.* Among them, *Blautia, Allobaculum, Prevotella, Bacteroides,* and *Butyricimonas* are relatively abundant and are capable of producing short-chain fatty acid.

Therefore, when administered to a subject, berberine is capable of enriching the short-chain fatty acid-producing bacteria and reducing endotoxin-producing bacteria in gut microbiota population.

### Berberine Increases Gut Short-Chain Fatty Acid Level in Rats

### Fed Normal Diet or High Fat Diet

The levels of short-chain fatty acid (including acetic acid, propionic acid, butyric acid, valeric acid, isobutyric acid, isovaleric acid, etc.) in rat feces were assayed by gas chromatography. The result demonstrated that oral administration of 100mg/kg body weight may increase the level of gut short-chain fatty acid in rats fed with normal diet or high fat diet. The effect on the levels of acetic acid and propionic acid are especially pronounced (FIGURE 3). Therefore, berberine is capable of increasing gut short-chain fatty acid level in rats fed normal diet or high fat diet.

### Berberine Reduces Obesity Phenotype of Rats

The body weight of all four groups of rats were monitored during the duration of the experiments and analyzed. The results demonstrated that, after 18 weeks of high fat diet, the HFD group has significantly higher body weight than the normal diet group (P<0.01); intragastric administration of berberine at the dosage of 100mg/kg effectively limits rat body weight growth, especially for rats fed high fat diet. The result is especially surprising that during the entire experimental process, the body weight of the HFD+BBR group is limited to a level similar to that of normal diet group, displaying no statistically significant difference (P>0.05). Berberine also influences, to a certain degree, the body weight of rats fed a normal diet (FIGURE 4).

At the end of experiment, animals were euthanized. Fasting body weight, epididymal fat weight, and perirenal fat weight were measured. Adiposity index ([epididymal fat weight + perirenal fat] / fasting weight × 100) is shown in FIGURE 4B. After 18 weeks of high fat diet, the adiposity index of the HFD group is significantly higher than that of NCD, and berberine treatment significantly reduces adiposity index. Moreover, neither high fat diet nor berberine had significant effect on liver and pancreas, indicating surprisingly that long term use of berberine has no obvious side effect to rat's normal physiological function. Results of the calorie intake of these rats demonstrated that oral administration of berberine at 100mg/kg body weight has a significant inhibitory effect on rat food intake, especially on rats fed a high fat diet (Figure 4C).

### Berberine Reduces Insulin Insensitivity in Rats Fed a Normal Diet or High Fat Diet

The fasting blood glucose (FBG) and fasting insulin in serum (FINS) were measured for all four experimental groups of rats. Rats fed a HFD for 18 months had a significant higher level of FBG than that of NCD. Surprisingly, berberine effectively reduced FBG in NCD and HFD rats, especially in HFD rats where the FBG reduction was significant (P<0.05) (FIGURE 5A). The result of berberine effect on insulin level is shown in FIGURE 5B. The FINS of rat fed a HFD was significantly higher than that of NCD group; however, after 18 month of berberine intervention at the level of 100mg/kg body weight, FINS level was significantly reduced even in rats fed a HFD (HFD+BBR), to reach a level comparable to that of normal diet. FIGURE 5C shows the result of HOMA insulin resistance index to evaluate the insulin resistance status in rats, which demonstrates that after 18 weeks of HFD induction, rats formed apparent insulin resistance; yet berberine intervention at 100mg/kg body weight prevented the formation of insulin resistance (P<0.05).

In order to further examine insulin sensitivity, oral glucose tolerance test and intraperitoneal injection of insulin tolerance test were conducted, and the results are shown in FIGURE 5D, and 5E, respectively. Consistent with the FBG and FINS result, after 18 weeks of high fat diet inducement, oral glucose tolerance and intraperitoneal injection of insulin tolerance were significantly damaged, meanwhile, berberine intervention at 100mg/kg body weight significantly prevented the loss of glucose tolerance and insulin tolerance, demonstrating the berberine may play an important role in improving glucose metabolism.

### Berberine Reduces Systemic inflammation Level in Rats

### Fed a High Fat Diet

In order to evaluate the systemic inflammation levels of all four experimental groups of rats, the serum levels of lipopolysaccharide (LPS)-binding protein (LBP), monocyte chemoattractant protein-1 (MCP-1), leptin, and adiponectin were measured, and results are shown in FIGURE 6. The experiments demonstrates that HFD significantly increased LBP level in serum; but administering berberine at 100mg/kg significantly abrogated the increase of serum LBP (P<0.05, FIGURE 6A).

MCP-1 is a pro-inflammatory cytokine that functions in chemotaxis and activation of monocytes/macrophages. Occurrence and development of many inflammation-related diseases are closely related to MCP-1, including atherosclerosis, obesity, type 2 diabetes, arthritis, sepsis, and chronic bacterial infection. The results from the experiment demonstrated that, in the process of the gradual onset of obesity and insulin resistance in rat induced by HFD, MCP-1 level is gradually elevated; however, after intervention with berberine administering, MCP-1 level was significantly reduced, and, surprisingly, was even lower than that of NCD group (FIGURE 6B).

Leptin is a hormone secreted by adipose tissue, and broadly participates in lipid, glucose, and energy metabolism. The results from the experiment demonstrated that serum leptin level in HFD group is significantly higher than that of NCD group (P<0.01); however, berberine significantly reduced the serum leptin level in rat, especially in rats fed a HFD (P<0.05, FIGURE 6C).

The adiponectin levels in all four groups were analyzed. The results show that the adiponectin level normalized against body fat weight, was significantly lower in HFD group when compared to that of NCD group (P<0.001); however, berberine administration significantly increased adiponectin level in rats fed a HFD (P<0.01, FIGURE 6D). Therefore, berberine improves gut microbiota population, reduces LPB, MCP-1, and lpetin level, and increases adiponectin secretion.

**TABLE 1 16S rRNA gene V3 region sequence of those bacteria enriched by berberine.**

| **List** | **OUT Name** | **16S rRNA Gene V3 Region Sequence** |
|---|---|---|
| SEQ_ID_NO_1 | R_U00449939 | |
| SEQ_ID_NO_2 | R_U01131573 | |
| SEQ_ID_NO_3 | R_U00442991 | |
| SEQ_ID_NO_4 | R_U01131468 | |
| SEQ_ID_NO_5 | R_U00000076 | |
| SEQ_ID_NO_6 | R_U00000261 | |
| SEQ_ID_NO_7 | R_U00277049 | |
| SEQ_ID_NO_8 | R_U01140154 | |
| | | |
| SEQ_ID_NO_9 | R_U00797917 | |
| SEQ_ID_NO_10 | R_U01135802 | |
| SEQ_ID_NO_11 | R_U01199632 | |
| SEQ_ID_NO_12 | R_U01136954 | |
| SEQ_ID_NO_13 | R_U01156163 | |
| SEQ_ID_NO_14 | R_U00436427 | |
| SEQ_ID_NO_15 | R_U01161731 | |
| SEQ_ID_NO_16 | R_U00804243 | |
| | | |
| SEQ_ID_NO_17 | R_U00832647 | |
| SEQ_ID_NO_18 | R_U00013412 | |
| SEQ_ID_NO_19 | R_U00807079 | |
| SEQ_ID_NO_20 | R_U00005346 | |
| SEQ_ID_NO_21 | R_U00004086 | |
| SEQ_ID_NO_22 | R_U01130438 | |
| SEQ_ID_NO_23 | R_U01131459 | |
| SEQ_ID_NO_24 | R_U00436448 | |
| | | |
| SEQ_ID_NO_25 | R_U00474862 | |
| SEQ_ID_NO_26 | R_U00001388 | |
| SEQ_ID_NO_27 | R_U01185184 | |
| SEQ_ID_NO_28 | R_U00000528 | |
| SEQ_ID_NO_29 | R_U00000002 | |
| SEQ_ID_NO_30 | R_U00147566 | |
| SEQ_ID_NO_31 | R_U00000394 | |
| SEQ_ID_NO_32 | R_U00047397 | |
| | | |
| SEQ_ID_NO_33 | R_U00000552 | |
| SEQ_ID_NO_34 | R_U00441706 | |
| SEQ_ID_NO_35 | R_U00119974 | |
| SEQ_ID_NO_36 | R_U00104206 | |
| SEQ_ID_NO_37 | R_U00580795 | |
| SEQ_ID_NO_38 | R_U01129900 | |
| SEQ_ID_NO_39 | R_U00016467 | |
| SEQ_ID_NO_40 | R_U00000367 | |
| SEQ_ID_NO_41 | R_U00459481 | |
| SEQ_ID_NO_42 | R_U00000215 | |
| SEQ_ID_NO_43 | R_U00071963 | |
| SEQ_ID_NO_44 | R_U00000939 | |
| SEQ_ID_NO_45 | R_U00472681 | |
| SEQ_ID_NO_46 | R_U00000105 | |
| SEQ_ID_NO_47 | R_U00000279 | |
| SEQ_ID_NO_48 | R_U00022935 | |
| SEQ_ID_NO_49 | R_U00000550 | |
| SEQ_ID_NO_50 | R_U00009280 | |
| SEQ_ID_NO_51 | R_U00164237 | |
| | | |
| SEQ_ID_NO_52 | R_U00032911 | |
| SEQ_ID_NO_53 | R_U00040533 | |
| SEQ_ID_NO_54 | R_U01145869 | |
| SEQ_ID _NO_55 | R_U00211687 | |
| SEQ_ID_NO_56 | R_U01206474 | |
| SEQ_ID_NO_57 | R_U01219720 | |
| SEQ_ID_NO_58 | R_U01210669 | |
| SEQ_ID_NO_59 | R_U00001394 | |
| SEQ_ID_NO_60 | R_U00460521 | |
| SEQ_ID_NO_61 | R_U00831992 | |
| | | |
| SEQ_ID_NO_62 | R_U01143501 | |
| SEQ_ID_NO_63 | R_U00815791 | |
| SEQ_ID_NO_64 | R_U00043629 | |
| SEQ_ID_NO_65 | R_U00164098 | |
| SEQ_ID_NO_66 | R_U00000374 | |
| SEQ_ID_NO_67 | R_U00003296 | |
| SEQ_ID_NO_68 | R_U00027329 | |
| SEQ_ID_NO_69 | R_U00003420 | |
| SEQ_ID_NO_70 | R_U00436238 | |
| SEQ_ID_NO_71 | R_U00439751 | |
| SEQ_ID_NO_72 | R_0004741 | |
| SEQ_ID_NO_73 | R_U01160251 | |
| SEQ_ID_NO_74 | R_U01166410 | |
| SEQ_ID_NO_75 | R_U01169117 | |
| SEQ_ID_NO_76 | R_U01180557 | |
| SEQ_ID_NO_77 | R_U00804007 | |
| SEQ_ID_NO_78 | R_U01143008 | |
| SEQ_ID_NO_79 | R_U01145549 | |
| | | |
| SEQ_ID_NO_80 | R_U00808391 | |
| SEQ_ID_NO_81 | R_U01144224 | |
| SEQ_ID_NO_82 | R_U01214804 | |
| SEQ_ID_NO_83 | R_U01173110 | |
| SEQ_ID_NO_84 | R_U00305232 | |
| SEQ_ID_NO_85 | R_U00043735 | |
| SEQ_ID_NO_86 | R_U00000409 | |
| SEQ_ID_NO_87 | R_U00029718 | |
| | | |
| SEQ_ID_NO_88 | R_U00007185 | |
| SEQ_ID_NO_89 | R_U00011248 | |
| SEQ_ID_NO_90 | R_U01156257 | |
| SEQ_ID_NO_91 | R_U00808203 | |
| SEQ_ID_NO_92 | R_U00003073 | |
| SEQ ID_NO_93 | R_U00000235 | |

**TABLE 2 16S rRNA gene V3 region sequence of those bacteria suppressed/eliminated by berberine.**

| **List** | **OUT Name** | **16S rRNA Gene V3 Region Sequence** |
|---|---|---|
| SEQ ID_NO_94 | R_U00436195 | |
| SEQ_ID_NO_95 | R_U00101416 | |
| SEQ_ID_NO_96 | R_U00000407 | |
| SEQ_ID_NO_97 | R_U00800966 | |
| SEQ_ID_NO_98 | R_U01131982 | |
| SEQ ID_NO_99 | R_U00797839 | |
| SEQ_ID_NO_100 | R_U00003981 | |
| SEQ_ID_NO_101 | R_U00001404 | |
| | | |
| SEQ_ID_NO_102 | R_U00000052 | |
| SEQ_ID_NO_103 | R_U00000964 | |
| SEQ_ID_NO_104 | R_U00040690 | |
| SEQ_ID_NO_105 | R_U00823991 | |
| SEQ_ID_NO_106 | R_U00000804 | |
| SEQ_ID_NO_107 | R_U01155915 | |
| SEQ_ID_NO_108 | R_U00459850 | |
| SEQ_ID_NO_109 | R_U00798098 | |
| | | |
| SEQ_ID_NO_110 | R_U00009170 | |
| SEQ_ID_NO_111 | R_U01102153 | |
| SEQ_ID_NO_112 | R_U00006230 | |
| SEQ_ID_NO_113 | R_U00465344 | |
| SEQ_ID_NO_114 | R_U00001202 | |
| SEQ_ID_NO_115 | R_U00001384 | |
| SEQ_ID_NO_116 | R_U00802799 | |
| SEQ_ID_NO_117 | R_U00808453 | |
| | | |
| SEQ_ID_NO_118 | R_U00026711 | |
| SEQ_ID_NO_119 | R_U00000650 | |
| SEQ_ID_NO_120 | R_U00000839 | |
| SEQ_ID_NO_121 | R_U01157930 | |
| SEQ_ID_NO_122 | R_U00798420 | |
| SEQ_ID_NO_123 | R_U00001933 | |
| SEQ_ID_NO_124 | R_U00001491 | |
| SEQ_ID_NO_125 | R_U00006476 | |
| | | |
| SEQ_ID_NO_126 | R_U00001502 | |
| SEQ_ID_NO_127 | R_U00001504 | |
| SEQ_ID_NO_128 | R_U00000609 | |
| SEQ_ID_NO_129 | R_U00009261 | |
| SEQ_ID_NO_130 | R_U01136992 | |
| SEQ_ID_NO_131 | R_U00000497 | |
| SEQ_ID_NO_132 | R_U00000886 | |
| SEQ_ID_NO_133 | R_U01143292 | |
| | | |
| SEQ_ID_NO_134 | R_U00803188 | |
| SEQ_ID_NO_135 | R_U00008782 | |
| SEQ_ID_NO_136 | R U00001406 | |
| SEQ_ID_NO_137 | R_U00030880 | |
| SEQ_ID_NO_138 | R_U00450938 | |
| SEQ_ID_NO_139 | R_U00009763 | |
| SEQ_ID_NO_140 | R_U01155495 | |
| SEQ_ID_NO_141 | R_U00798694 | |
| | | |
| SEQ_ID_NO_142 | R_U00802360 | |
| SEQ_ID_NO_143 | R_U01162782 | |
| SEQ_ID_NO_144 | R_U00002709 | |
| SEQ_ID_NO_145 | R_U00005232 | |
| SEQ_ID_NO_146 | R_U0000305 | |
| SEQ_ID_NO_147 | R_00006821 | |
| SEQ_ID_NO_148 | R_01199405 | |
| SEQ_ID_NO_149 | R_00006150 | |
| | | |
| SEQ_ID_NO_150 | R_U00000206 | |
| SEQ_ID_NO_151 | R_U01150673 | |
| SEQ_ID_NO_152 | R_U00805811 | |
| SEQ_ID_NO_153 | R_U01199471 | |
| SEQ_ID_NO_154 | R_U00000068 | |
| SEQ_ID_NO_155 | R_U00005559 | |
| SEQ_ID_NO_156 | R_U00801961 | |
| SEQ_ID_NO_157 | R_U00000126 | |
| | | |
| SEQ_ID_NO_158 | R_U00164105 | |
| SEQ_ID_NO_159 | R_U00118078 | |
| SEQ_ID_NO_160 | R_U01148376 | |
| SEQ_ID_NO_161 | R_U00444002 | |
| SEQ ID_NO_162 | R_U01130396 | |
| SEQ_ID_NO_163 | R_U00011582 | |
| SEQ_ID_NO_164 | R_U00439888 | |
| SEQ_ID_NO_165 | R_U01163617 | |
| SEQ_ID_NO_166 | R_U00029306 | |
| SEQ_ID_NO_167 | R_U00797985 | |
| | | |
| SEQ_ID_NO_168 | R_U01173594 | |
| SEQ_ID_NO_169 | R_U00009282 | |
| SEQ_ID_NO_170 | R_U00165288 | |
| SEQ_ID_NO_171 | R_U00003481 | |
| SEQ_ID_NO_172 | R_U00003548 | |
| SEQ_ID_NO_173 | R_U00798472 | |
| SEQ_ID_NO_174 | R_U00804005 | |
| SEQ_ID_NO_175 | R_U00164814 | |
| SEQ_ID_NO_176 | R_U00507409 | |
| SEQ_ID_ NO_177 | R_U00009952 | |
| | | |
| SEQ_ID_NO_178 | R_U00832549 | |
| SEQ_ID_NO_179 | R_U00013476 | |
| SEQ_ID_NO_180 | R_U00000012 | |
| SEQ_ID_NO_181 | R_U00808065 | |
| SEQ_ID_NO_182 | R_U00816625 | |
| SEQ_ID_NO_183 | R_U00164605 | |
| SEQ_ID_NO_184 | R_U00232096 | |
| SEQ_ID_NO_185 | R_U00031502 | |
| SEQ_ID_NO_186 | R_U01130683 | |
| SEQ_ID_NO_187 | R_U01139355 | |
| SEQ_ID_NO_188 | R_U00851599 | |
| SEQ_ID_NO_189 | R_U00010047 | |
| SEQ_ID_NO_190 | R_U00164135 | |
| SEQ_ID_NO_191 | R_U00034050 | |
| SEQ_ID_NO_192 | R_U01139670 | |
| SEQ_ID_NO_193 | R_U00033670 | |
| SEQ_ID_NO_194 | R_U00033693 | |
| SEQ_ID_NO_195 | R_U00002583 | |
| SEQ_ID_NO_196 | R_U00044129 | |
| | | |
| SEQ_ID_NO_197 | R_U00042653 | |
| SEQ_ID_NO_198 | R_U00855328 | |
| SEQ_ID_NO_199 | R_U00164196 | |
| SEQ_ID_NO_200 | R_U00164256 | |
| SEQ_ID_NO_201 | R_U00003079 | |
| SEQ_ID_NO_202 | R_U00002404 | |
| SEQ_ID_NO_203 | R_U00804375 | |
| SEQ_ID_NO_204 | R_U00443872 | |
| SEQ_ID_NO_205 | R_U00000128 | |
| SEQ_ID_NO_206 | R_U00002777 | |
| | | |
| SEQ_ID_NO_207 | R_U00001166 | |
| SEQ_ID_NO_208 | R_U00002087 | |
| SEQ_ID_NO_209 | R_U00008098 | |
| SEQ_ID_NO_210 | R_U00000981 | |
| SEQ_ID_NO_211 | R_U00436292 | |
| SEQ_ID_NO_212 | R_U00002551 | |
| SEQ_ID_NO_213 | R-U00164297 | |
| SEQ_ ID_NO_214 | R_U00806937 | |
| SEQ_ID_NO_215 | R_U00002723 | |
| SEQ_ID_NO_216 | R_U00004878 | |
| | | |
| SEQ_ID_NO_217 | R_U01142982 | |
| SEQ_ID_NO_218 | R_U00806884 | |
| SEQ_ID_NO_219 | R_U00810531 | |
| SEQ_ID_NO_220 | R_U00000149 | |
| SEQ_ID_NO_221 | R_U00004501 | |
| SEQ_ID_NO_222 | R_U00132882 | |
| SEQ_ID_NO_223 | R_U00800387 | |
| SEQ_ID_NO_224 | R_U00000908 | |
| SEQ_ID_NO_225 | R_U00204386 | |
| SEQ_ID_NO_226 | R_U00799453 | |
| SEQ_ID_NO_227 | R_U00806071 | |
| SEQ_ID_NO_228 | R_U01132907 | |
| SEQ_ID_NO_229 | R_U00463337 | |
| SEQ_ID_NO_230 | R_U00812994 | |
| SEQ_ID_NO_231 | R_U00010018 | |
| SEQ_ID_NO_232 | R_U00164720 | |
| SEQ_ID_NO_233 | R_U00004418 | |
| SEQ_ID_NO_234 | R_U00208153 | |
| SEQ_ID_NO_235 | R_U00030870 | |
| | | |
| SEQ_ID_NO_236 | R_U00837797 | |
| SEQ_ID_NO_237 | R_U00449804 | |
| SEQ_ID_NO_238 | R_U00436332 | |
| SEQ_ID_NO_239 | R_U00003435 | |
| SEQ_ID_NO_240 | R_U00198673 | |
| SEQ_ID_NO_241 | R_U00002720 | |
| SEQ_ID_NO_242 | R_U00195707 | |
| SEQ_ID_NO_243 | R_U00002686 | |
| SEQ_ID_NO_244 | R_U00008959 | |
| SEQ_ID_NO_245 | R_U00034351 | |
| | | |
| SEQ_ID_NO_246 | R_U00437009 | |
| SEQ_ID_NO_247 | R_U00032486 | |
| SEQ_ID_NO_248 | R_U00000999 | |
| SEQ_ID_NO_249 | R_U00002201 | |
| SEQ_ID_NO_250 | R_U00001891 | |
| SEQ_ID_NO_251 | R_U00803665 | |
| SEQ_ID_NO_252 | R_U00027111 | |
| SEQ_ID_NO_253 | R_U00454157 | |
| SEQ_ID_NO_254 | R_U00080174 | |
| SEQ_ID_NO_255 | R_U00803302 | |
| | | |
| SEQ_ID_NO_256 | R_U00032208 | |
| SEQ_ID_NO_257 | R_U00001116 | |
| SEQ_ID_NO_258 | R_U00807704 | |
| SEQ_ID_NO_259 | R_U00000272 | |
| SEQ_ID_NO_260 | R_U00805277 | |
| SEQ_ID_NO_261 | R_U01136238 | |
| SEQ_ID_NO_262 | R_U00848639 | |
| SEQ_ID_NO_263 | R_U00004249 | |
| SEQ_ID_NO_264 | R_U00012096 | |
| | | |
| SEQ_ID_NO_265 | R_U01152122 | |
| SEQ_ID_NO_266 | R_U01123199 | |
| SEQ_ID_NO_267 | R_U00000737 | |
| SEQ_ID_NO_268 | R_U00822040 | |

In the above detailed description, reference is made to the accompanying drawings, which form a part hereof. In the drawings, similar symbols typically identify similar components, unless context dictates otherwise. The illustrative embodiments described in the detailed description, drawings, and claims are not meant to be limiting. Other embodiments may be used, and other changes may be made, without departing from the scope of the appended claims. The present disclosure is not to be limited in terms of the particular examples described in this application, which are intended as illustrations of various aspects. Many modifications and variations can be made, as will be apparent to those skilled in the art. Functionally equivalent methods and apparatuses within the scope of the disclosure, in addition to those enumerated herein, will be apparent to those skilled in the art from the foregoing descriptions. Such modifications and variations are intended to fall within the scope of the appended claims. The present disclosure is to be limited only by the terms of the appended claims, along with the full scope of equivalents to which such claims are entitled. It is to be understood that this disclosure is not limited to particular methods, reagents, compounds, compositions or biological systems, which can, of course, vary. It is also to be understood that the terminology used herein is for the purpose of describing particular embodiments only, and is not intended to be limiting.

With respect to the use of substantially any plural and/or singular terms herein, those having skill in the art can translate from the plural to the singular and/or from the singular to the plural as is appropriate to the context and/or application. The various singular/plural permutations may be expressly set forth herein for sake of clarity.

It will be understood by those within the art that, in general, terms used herein, and especially in the appended claims (*e.g*., bodies of the appended claims) are generally intended as "open" terms (*e.g*., the term "including" should be interpreted as "including but not limited to," the term "having" should be interpreted as "having at least," the term "includes" should be interpreted as "includes but is not limited to," etc.). It will be further understood by those within the art that if a specific number of an introduced claim recitation is intended, such an intent will be explicitly recited in the claim, and in the absence of such recitation no such intent is present. For example, as an aid to understanding, the following appended claims may contain usage of the introductory phrases "at least one" and "one or more" to introduce claim recitations. However, the use of such phrases should not be construed to imply that the introduction of a claim recitation by the indefinite articles "a" or "an" limits any particular claim containing such introduced claim recitation to embodiments containing only one such recitation, even when the same claim includes the introductory phrases "one or more" or "at least one" and indefinite articles such as "a" or "an" (*e.g*., "a" and/or "an" should be interpreted to mean "at least one" or "one or more"); the same holds true for the use of definite articles used to introduce claim recitations. In addition, even if a specific number of an introduced claim recitation is explicitly recited, those skilled in the art will recognize that such recitation should be interpreted to mean at least the recited number (*e.g*., the bare recitation of "two recitations," without other modifiers, means at least two recitations, or two or more recitations). Furthermore, in those instances where a convention analogous to "at least one of A, B, and C, etc." is used, in general such a construction is intended in the sense one having skill in the art would understand the convention (*e.g.*, " a system having at least one of A, B, and C" would include but not be limited to systems that have A alone, B alone, C alone, A and B together, A and C together, B and C together, and/or A, B, and C together, etc.). In those instances where a convention analogous to "at least one of A, B, or C, etc." is used, in general such a construction is intended in the sense one having skill in the art would understand the convention (*e.g*., " a system having at least one of A, B, or C" would include but not be limited to systems that have A alone, B alone, C alone, A and B together, A and C together, B and C together, and/or A, B, and C together, etc.). It will be further understood by those within the art that virtually any disjunctive word and/or phrase presenting two or more alternative terms, whether in the description, claims, or drawings, should be understood to contemplate the possibilities of including one of the terms, either of the terms, or both terms. For example, the phrase "A or B" will be understood to include the possibilities of "A" or "B" or "A and B."

In addition, where features or aspects of the disclosure are described in terms of Markush groups, those skilled in the art will recognize that the disclosure is also thereby described in terms of any individual member or subgroup of members of the Markush group.

As will be understood by one skilled in the art, for any and all purposes, such as in terms of providing a written description, all ranges disclosed herein also encompass any and all possible subranges and combinations of subranges thereof. Any listed range can be easily recognized as sufficiently describing and enabling the same range being broken down into at least equal halves, thirds, quarters, fifths, tenths, etc. As a non-limiting example, each range discussed herein can be readily broken down into a lower third, middle third and upper third, etc. As will also be understood by one skilled in the art all language such as "up to," "at least," and the like include the number recited and refer to ranges which can be subsequently broken down into subranges as discussed above. Finally, as will be understood by one skilled in the art, a range includes each individual member. Thus, for example, a group having 1-3 cells refers to groups having 1, 2, or 3 cells. Similarly, a group having 1-5 cells refers to groups having 1, 2, 3, 4, or 5 cells, and so forth.

From the foregoing, it will be appreciated that various embodiments of the present disclosure have been described herein for purposes of illustration, and that various modifications may be made. Accordingly, the various embodiments disclosed herein are not intended to be limiting, with the true scope being indicated by the following claims.

## Claims

1. Berberine for use in a method for improving gut microbiota population in a subject having obesity or insulin resistance, wherein improving the gut microbiota population is **characterized by** selectively increasing a first gut microbiota population while simultaneously decreasing a second gut microbiota population to increase short-chain fatty acid level in the subject or reduce subject insulin resistance, the method comprising administering to the subject berberine at a dosage of 100mg/Kg body weight,
wherein the first gut microbiota population comprises *Allobaculum, Bacteroides, Blautia, Butyricicoccus, Dorea, Holdemania, Lawsonia, Parabacteroides, Phascolarctobacterium, Sedimentibacter,* or a combination thereof, and
wherein the second gut microbiota population comprises *Alistipes, Anaeroplasma, Barnesiella, Bifidobacterium, Butyricimonas, Butyrivibrio, Coprococcus, Fastidiosipila, Helicobacter, Hespellia, Marvinbryantia, Oribacterium, Oscillibacter, Prevotella, Roseburia, Ruminococcus,* or a combination thereof.

2. The berberine for use of Claim 1, wherein the berberine is prepared as a food, a drink, a supplement, or a pharmaceutical formulation.

3. The berberine for use of Claim 1, wherein the method for improving the gut microbiota population comprises administering the berberine to a subject at a dosage of 100 mg/body weight and with once a day administration for at least two weeks.

4. The berberine for use of Claim 1, wherein the first gut microbiota population comprises a bacterium whose V3 region of 16S rRNA gene sequence has at least 95% similarity with a nucleic acid sequence selected from a group consisting of SEQ ID NO: 1-93.

5. The berberine for use of Claim 1, wherein the first gut microbiota population comprises a bacterium whose V3 region of 16S rRNA gene sequence has at least 80% similarity with a nucleic acid sequence selected from a group consisting of SEQ ID NO: 1-93.

6. The berberine for use of Claim 1, wherein the second gut microbiota population comprises a bacterium whose V3 region of 16S rRNA gene sequence has at least 95% or 80% similarity with a nucleic acid sequence selected from a group consisting of SEQ ID NO: 94-268.

## Patentansprüche

1. Berberin zur Verwendung in einem Verfahren zum Verbessern von Darmmikrobiota-Population bei einem Subjekt, das Adipositas oder Insulinresistenz aufweist, wobei ein Verbessern der Darmmikrobiota-Population **gekennzeichnet ist durch** selektives Erhöhen einer ersten Darmmikrobiota-Population, während gleichzeitig eine zweite Darmmikrobiota-Population verringert wird, um einen Spiegel von kurzkettiger Fettsäure bei dem Subjekt zu erhöhen oder Insulinresistenz des Subjekts zu reduzieren, das Verfahren umfassend ein Verabreichen von Berberin in einer Dosierung von 100 mg/kg Körpergewicht an das Subjekt,
wobei die erste Darmmikrobiota-Population *Allobaculum, Bacteroides, Blautia, Butyricicoccus, Dorea, Holdemania, Lawsonia, Parabacteroides, Phascolarctobacterium, Sedimentibacter* oder eine Kombination davon umfasst, und
wobei die zweite Darmmikrobiota-Population *Alistipes, Anaeroplasma, Barnesiella, Bifidobacterium, Butyricimonas, Butyrivibrio, Coprococcus, Fastidiosipila, Helicobacter, Hespellia, Marvinbryantia, Oribacterium, Oscillibacter, Prevotella, Roseburia, Ruminococcus* oder eine Kombination davon umfasst.

2. Berberin zur Verwendung gemäß Anspruch 1, wobei das Berberin als ein Nahrungsmittel, ein Getränk, eine Ergänzung oder eine pharmazeutische Formulierung hergestellt ist.

3. Berberin zur Verwendung gemäß Anspruch 1, wobei das Verfahren zur Verbesserung der Darmmikrobiota-Population ein Verabreichen des Berberins an ein Subjekt in einer Dosierung von 100 mg/Körpergewicht und mit einmal täglicher Verabreichung über mindestens zwei Wochen umfasst.

4. Berberin zur Verwendung gemäß Anspruch 1, wobei die erste Darmmikrobiota-Population ein Bakterium umfasst, dessen V3-Region von 16S rRNA-Gensequenz mindestens 95% Ähnlichkeit mit einer Nukleinsäuresequenz aufweist, die aus einer Gruppe ausgewählt ist, die aus SEQ ID NO: 1-93 besteht.

5. Berberin zur Verwendung gemäß Anspruch 1, wobei die erste Darmmikrobiota-Population ein Bakterium umfasst, dessen V3-Region von 16S rRNA-Gensequenz mindestens 80% Ähnlichkeit mit einer Nukleinsäuresequenz aufweist, die aus einer Gruppe ausgewählt ist, die aus SEQ ID NO: 1-93 besteht.

6. Berberin zur Verwendung gemäß Anspruch 1, wobei die zweite Darmmikrobiota-Population ein Bakterium umfasst, dessen V3-Region von 16S rRNA-Gensequenz mindestens 80% Ähnlichkeit mit einer Nukleinsäuresequenz aufweist, die aus einer Gruppe ausgewählt ist, die aus SEQ ID NO: 94-268 besteht.

## Revendications

1. Berbérine pour utilisation dans un procédé pour améliorer la population de microbiote intestinal chez un sujet souffrant d'obésité ou présentant une résistance à l'insuline, dans laquelle l'amélioration de la population de microbiote intestinal est **caractérisée par** l'augmentation sélective d'une première population de microbiote intestinal tout en diminuant simultanément une deuxième population de microbiote intestinal pour augmenter le niveau d'acide gras à chaîne courte chez le sujet ou réduire la résistance à l'insuline du sujet, le procédé comprenant l'administration de berbérine au sujet à une dose de 100 mg/kg de poids corporel,
dans laquelle la première population de microbiote intestinal comprend *Allobaculum, Bacteroides, Blautia, Butyricicoccus, Dorea, Holdemania, Lawsonia, Parabacteroides, Phascolarctobacterium, Sedimentibacter,* ou une combinaison de ceux-ci, et
dans laquelle la deuxième population de microbiote intestinal comprend *Alistipes, Anaeroplasma, Barnesiella, Bifidobacterium, Butyricimonas, Butyrivibrio, Coprococcus, Fastidiosipila, Helicobacter, Hespellia, Marvinbryantia, Oribacterium, Oscillibacter Prevotella, Roseburia, Ruminococcus,* ou une combinaison de ceux-ci.

2. La berbérine pour utilisation selon la revendication 1, dans laquelle la berbérine est préparée sous forme d'aliment, de boisson, de complément, ou de formulation pharmaceutique.

3. La berbérine pour utilisation selon la revendication 1, dans laquelle le procédé pour améliorer la population de microbiote intestinal comprend l'administration de la berbérine à un sujet à une dose de 100 mg/poids corporel et avec une administration par jour pendant au moins deux semaines.

4. La berbérine pour utilisation selon la revendication 1, dans laquelle la première population de microbiote intestinal comprend une bactérie dont la région V3 de la séquence de gène de l'ARNr 16S a une similarité d'au moins 95 % avec une séquence d'acide nucléique choisie dans un groupe constitué par SEQ ID NO : 1-93.

5. La berbérine pour utilisation selon la revendication 1, dans laquelle la première population de microbiote intestinal comprend une bactérie dont la région V3 de la séquence de gène de l'ARNr 16S a une similarité d'au moins 80 % avec une séquence d'acide nucléique choisie dans un groupe constitué par SEQ ID NO : 1-93.

6. La berbérine pour utilisation selon la revendication 1, dans laquelle la deuxième population de microbiote intestinal comprend une bactérie dont la région V3 de la séquence de gène de l'ARNr 16S a une similarité d'au moins 95 % ou 80 % avec une séquence d'acide nucléique choisie dans un groupe constitué par SEQ ID NO : 94-268.
